(19) Europäisches Patentamt / European Patent Office / Office européen des brevets

(11) **EP 3 943 947 A1**

(12) **EUROPEAN PATENT APPLICATION**

(43) Date of publication:
**26.01.2022 Bulletin 2022/04**

(21) Application number: **20382658.1**

(22) Date of filing: **22.07.2020**

(51) International Patent Classification (IPC):
**G01N 33/68** $^{(2006.01)}$   **G01N 33/543** $^{(2006.01)}$
**C07K 14/31** $^{(2006.01)}$   **A61P 25/28** $^{(2006.01)}$
**A61K 38/00** $^{(2006.01)}$

(52) Cooperative Patent Classification (CPC):
**A61P 25/28; A61K 38/00; G01N 33/54373;
G01N 33/6896**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Universitat Autònoma de Barcelona
  08193 Bellaterra (ES)**
• **Universidad De Zaragoza
  50009 Zaragoza (ES)**

(72) Inventors:
• **Ventura Zamora, Salvador
  08193 Bellaterra (Cerdanyola del Valles) (ES)**
• **Pallarès Goitiz, Irantzu
  08193 Bellaterra (Cerdanyola del Valles) (ES)**
• **Santos Suárez, Jaime
  08193 Bellaterra (Cerdanyola del Valles) (ES)**
• **Cremades Casasin, Nunilo
  5009 Zaragoza (Zaragoza) (ES)**
• **Gracia González, Pablo José
  5009 Zaragoza (Zaragoza) (ES)**

(54) **INHIBITORS OF ALPHA-SYNUCLEIN AGGREGATION AND USES THEREOF**

(57)    The present invention relates to inhibitors of α-synuclein aggregation which are characterized by comprising an amphipathic α-helical peptide. The invention further relates to therapeutic and diagnostic uses of such inhibitors as well as corresponding plasmonic sensors, kits, and pharmaceutical compositions.

Figure 1

| | Monomer | Oligomer type A* | Oligomer type B* | Fibrils |
|---|---|---|---|---|
| **Number of monomers** | 1 | ~30† | ~30† | >100† |
| **Anionic character** | ~9 | ~-270† | ~-270† | >-900 |
| **Relative hydrophobicity** | Low | Low | High | Medium |
| **Secondary structure** | Disordered | Disordered | β-sheet | β-sheet |

EP 3 943 947 A1

SEQUENCE LISTING

<110>    Universitat Autònoma de Barcelona

<120>    Inhibitors of a-synuclein aggregation and uses thereof

<130>    N-15 808

<160>    9

<170>    PatentIn version 3.5

<210>    1
<211>    140
<212>    PRT
<213>    Homo sapiens

<400>    1

Met Asp Val Phe Met Lys Gly Leu Ser Lys Ala Lys Glu Gly Val Val
1               5                   10                  15


Ala Ala Ala Glu Lys Thr Lys Gln Gly Val Ala Glu Ala Ala Gly Lys
            20                  25                  30


Thr Lys Glu Gly Val Leu Tyr Val Gly Ser Lys Thr Lys Glu Gly Val
            35                  40                  45


Val His Gly Val Ala Thr Val Ala Glu Lys Thr Lys Glu Gln Val Thr
        50                  55                  60


Asn Val Gly Gly Ala Val Val Thr Gly Val Thr Ala Val Ala Gln Lys
65                  70                  75                  80


Thr Val Glu Gly Ala Gly Ser Ile Ala Ala Ala Thr Gly Phe Val Lys
                85                  90                  95


Lys Asp Gln Leu Gly Lys Asn Glu Glu Gly Ala Pro Gln Glu Gly Ile
            100                 105                 110


Leu Glu Asp Met Pro Val Asp Pro Asp Asn Glu Ala Tyr Glu Met Pro
            115                 120                 125


Ser Glu Glu Gly Tyr Gln Asp Tyr Glu Pro Glu Ala
        130                 135                 140


<210>    2
<211>    22
<212>    PRT
<213>    Artificial Sequence

<220>
<223>    synthetic

22

<400> 2

Met Glu Phe Val Ala Lys Leu Phe Lys Phe Phe Lys Asp Leu Leu Gly
1               5               10              15

Lys Phe Leu Gly Asn Asn
            20


<210> 3
<211> 22
<212> PRT
<213> Artificial Sequence

<220>
<223> synthetic

<400> 3

Met Glu Phe Val Ala Lys Leu Phe Pro Phe Pro Lys Asp Leu Leu Gly
1               5               10              15

Lys Phe Leu Gly Asn Asn
            20


<210> 4
<211> 22
<212> PRT
<213> Artificial Sequence

<220>
<223> synthetic

<400> 4

Leu Glu Leu Leu Ala Lys Leu Leu Lys Leu Leu Lys Asp Leu Leu Gly
1               5               10              15

Lys Leu Leu Gly Asn Asn
            20


<210> 5
<211> 19
<212> PRT
<213> Artificial Sequence

<220>
<223> synthetic

<400> 5

Leu Glu Leu Leu Ala Lys Leu Leu Lys Leu Leu Lys Asp Leu Leu Gly
1               5               10              15

Lys Leu Leu

<210> 6
<211> 19
<212> PRT
<213> Artificial Sequence

<220>
<223> synthetic

<400> 6

Leu Glu Leu Leu Glu Lys Leu Leu Lys Leu Leu Lys Asp Leu Leu Lys
1               5                   10                  15

Lys Leu Leu


<210> 7
<211> 19
<212> PRT
<213> Artificial Sequence

<220>
<223> synthetic

<400> 7

Leu Glu Leu Leu Glu Glu Leu Leu Lys Leu Leu Glu Asp Leu Leu Lys
1               5                   10                  15

Lys Leu Leu


<210> 8
<211> 37
<212> PRT
<213> Artificial Sequence

<220>
<223> synthetic

<400> 8

Leu Leu Gly Asp Phe Phe Arg Lys Ser Lys Glu Lys Ile Gly Lys Gly
1               5                   10                  15

Phe Lys Arg Ile Val Gln Arg Leu Lys Asp Phe Leu Arg Asn Leu Val
            20                  25                  30

Pro Arg Thr Glu Ser
            35


<210> 9
<211> 72
<212> PRT
<213> Artificial Sequence

24

```
<220>
<223>  synthetic

<400>  9

Met Asp Val Phe Met Lys Gly Leu Ser Met Ala Lys Glu Gly Val Val
1               5                  10                  15


Ala Ala Ala Glu Lys Thr Lys Gln Gly Val Thr Glu Ala Ala Glu Lys
            20                  25                  30


Thr Lys Glu Gly Val Leu Tyr Val Gly Ser Lys Thr Arg Glu Gly Val
            35                  40                  45


Val Gln Gly Val Ala Ser Val Ala Glu Lys Thr Lys Glu Gln Ala Ser
        50                  55                  60


His Leu Gly Gly Ala Val Phe Ser
65                  70
```

**Description**

**FIELD OF THE INVENTION**

The present invention generally relates to inhibitors of α-synudein aggregation which are characterized by comprising an amphipathic α-helical peptide. The invention further relates to therapeutic and diagnostic uses of such inhibitors as well as corresponding plasmonic sensors, kits, and pharmaceutical compositions.

**BACKGROUND OF THE INVENTION**

Neurological disorders are presently the leading source of disability on the globe, among which Parkinson's disease (PD) is the fastest growing neurological disorder with more than 10 million people suffering from PD worldwide. The average age of PD onset is 60 with incidence increasing significantly with age. Driven principally by aging the number of PD patients is projected to further increase significantly, putting a lot of pressure in public health systems and societies as a whole.

α-Synudein (also designated 'αS' or 'α-Syn' herein) is a highly soluble, intrinsically disordered protein whose aggregation into amyloid fibrils lies behind the onset and progression of several neurodegenerative disorders (collectively referred to as α-synucleinopathies) including PD (Spillantini et al., 1999). αS is 140 amino acid residues in length and primarily composed of three regions: an amino terminus responsible for membrane interaction, a 30 disordered acidic carboxyl-terminal tail, and the hydrophobic motif (amino acid residues 65-90), wherein the latter is critical for protein aggregation and is also commonly referred to as the non-amyloid-β component of Alzheimer Disease amyloid plaques (NAC).

Interfering with αS aggregation and/or suppressing αS aggregate associated toxicity is generally considered as a promising molecular strategy for the treatment of these debilitating disorders (Pujols et al., 2020). Yet, it has been challenging to design new molecular entities that specifically target αS pathogenic assemblies due to the intrinsic complexity and heterogeneity of αS aggregation. This restriction forced the use of high-throughput methodologies to screen large libraries of compounds that resulted in the identification of promising candidates (Pujols et al., 2018; Peña-Diaz et al., 2019). However, such blinded selection often results in molecules with non-defined mechanism of action, precluding further rational evolution or applications. Moreover, some of these molecules act by interfering with functional species of αS (monomer) or kidnaping the protein into non-amyloid off-pathway aggregates; which are not the ideal strategies.

In line with such shortcomings, the development of early detection systems based on the detection of pathogenic forms of αS has been hindered by the absence of specific and sensitive molecules that solely target those species. To date, there is no definite, sensitive and predictive laboratory test that can identify PD individuals well before they show the clinical manifestation. Thus, current PD diagnosis is clinical in nature. In the clinical setting, PD is commonly missed or misdiagnosed since many symptoms of PD are also common to other diseases, both neurodegenerative and non-neurodegenerative. There is evidence showing that nearly half (47%) of PD diagnoses are incorrect when performed in the primary care setting. The diagnosis and treatment of PD typically occurs when the disease has already progressed to a relatively advanced stage in which motor symptoms are clearly evident and substantial neurophysiological damage has already taken place. At this point, any possibility of delaying disease progression may already be out of reach.

Early diagnosis and treatment are paramount to reducing the risk of disease progression, limiting the effects of PD on quality of life, and potentially lowering long-term treatment costs. The development of a simple conclusive diagnostic test would revolutionize clinical care and prevent PD misdiagnosis. It will also impulse research significantly enabling researchers to better understand the disease process and to develop and test treatments that may slow or stop progression.

In recent years, a large body of evidence describing αS aggregation pathways has led to the identification of low-molecular weight soluble oligomers with pivotal roles in the etiopathogenesis of α-synucleinopathies (Winner et al., 2011, Fusco et al., 2017). These oligomeric species, typically populated during the early events of αS aggregation, are more toxic than end-stage amyloid fibrils and retain the ability to nucleate the formation of full-length amyloid fibrils (Cremades et al., 2012). Growing evidence suggest that such toxic oligomers are the primary pathological agent behind gain-of-toxicity events in αS aggregation, while αS fibrils are being further associated with self-propagation, pointing to a critical role in disease spreading (Froula et al., 2019). The inventors recently identified two distinct types of αS oligomers formed during *in vitro* fibrillation by means of single-molecule techniques: non-toxic disordered oligomers (also known as type-A oligomers) that undergo a structural conversion to more stable and compact β-sheet enriched species (also referred as type-B oligomers) that retain an intrinsic cytotoxicity (Cremades et al., 2012). This novel assemblies offer a new therapeutic target that can be exploited for both therapeutic and diagnosis approaches.

There is evidence that a-Syn aggregates are elevated in the cerebrospinal fluid (CSF) of PD patients as compared to non-PD controls, which indicate that the levels of these species in this biofluid can be used as a biomarker for PD (Shahnawaz et al., 2017). There are PD diagnostic tests based on a-Syn aggregates detection which are currently in late development stages, the technology applied can be segmented into 3 different types:

- ELISA detection of a-Syn aggregates in CSF which is performed using antibodies that recognize also soluble a-Syn, which indeed is much more abundant, and thus requires complex sample manipulation;

- Protein-Misfolding Cyclic Amplification (PMCA) and Real-Time Quaking-Induced Conversion (RT-QuIC), two ultrasensitive protein amplification assays for the detection of misfolded protein aggregates; originally developed for the diagnosis of prion diseases.

The results obtained with these techniques are really encouraging in terms of patients identification and stratification. However, they also exhibit important problems for their implementation for routine diagnostic. The most important one is that it is impossible to know which is the specific a-Syn species that is amplified in the reaction, and thus the molecular biomarker on which the diagnostic is based. Moreover, the assays display low reproducibility between different laboratories and they are long. It is therefore highly desirable to provide a new technology for early detection of $\alpha$-synucleinopathies such as PD that uses a real-time, highly-specific and highly-sensitive assay requiring minimal volumes of biological sample (e.g., CSF). Such a technology might have a large impact on enabling the biochemical diagnosis of $\alpha$-synudeinopathies, perhaps even pre-symptomatically. As a diagnostic tool, it will assist clinicians in diagnosing and monitoring the disease, in providing prognoses and in anticipating treatment responses of the patients.

Likewise, it is highly desirable to provide new therapeutics platform for treatment of $\alpha$-synucleinopathies which is based on a defined mechanism of action, thus allowing for further rational evolution or applications, and provides therapeutics which do not interfere with the normal function of $\alpha$S (monomer).

## SUMMARY OF THE INVENTION

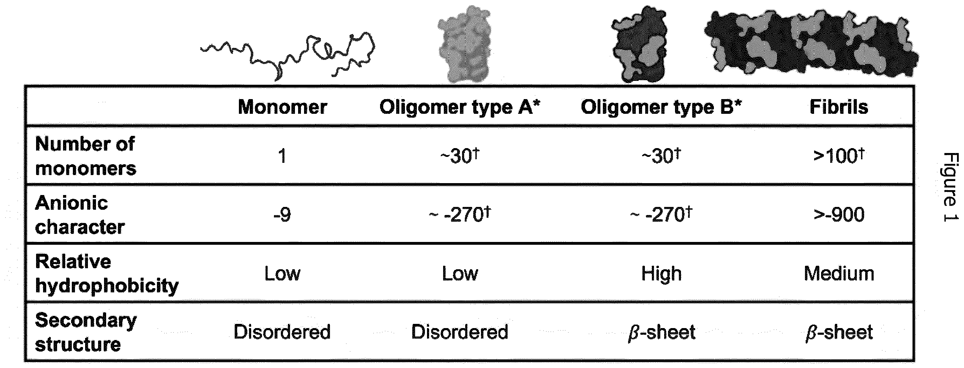

The different $\alpha$-synudein species (monomers, non-toxic oligomers, toxic oligomers, and fibrils) have different biophysical and structural features which apparently have distinct implications in the pathogenesis of $\alpha$-synudeinopathies. The inventors therefore started from the assumption that the particular idiosyncrasy of the different $\alpha$-synudein species give rise to very particular aberrant physicochemical features and that such peculiarities can be exploited to engineer a selective binder for toxic $\alpha$-synuclein oligomers and $\alpha$-synudein fibrils. Towards that end, the inventors dissected the main differential features of each $\alpha$S species as shown in Figure 1. Briefly, toxic $\alpha$S oligomers ("type-B oligomers") and $\alpha$S fibrils share two common features:

(i) Displaying large hydrophobic surfaces exposed to the solvent. Such hydrophobicity is responsible for inducing cellular toxicity and driving subsequent fibrillation (Chen et al., 2015, Fusco et al., 2017)

(ii) Possessing an extremely anionic character derived from the stacking of several copies of $\alpha$S (net charge -9).

Overall, toxic $\alpha$S species can be described as highly hydrophobic and anionic supramolecular structures. On that bases, the inventors hypothesized that the hydrophobic patches exposed by these pathogenic agents, embedded in an anionic environment, can shape a potential binding surface for a complementary molecule; ideally an amphipathic and cationic entity. The inventors found that a short $\alpha$-helical peptide is an ideal scaffold to concatenate such properties, thus allowing the peptide to bind toxic $\alpha$S oligomers and fibrils, and inhibit $\alpha$S aggregation.

In a first step, the inventors identified PSM$\alpha$3, a 22 amino acid extracellular amphipathic cationic peptide that fulfilled their desired characteristics (Marinelli et al., 2016). The inventors examined the binding specificity and $\alpha$S aggregation-inhibitory activity of PSM$\alpha$3 and of a non-$\alpha$-helical variant therefore (termed 'dPSM$\alpha$3' herein). The inventors found that PSM$\alpha$3 (but not dPSM$\alpha$3) is able to bind pathogenic $\alpha$S species (toxic $\alpha$S oligomers and $\alpha$S fibrils) specifically and with high affinity in the picomolar to nanomolar range, and proved that this interaction resulted in a substoichiometry inhibition of the $\alpha$S aggregation and in reduced cytotoxicity of toxic $\alpha$S oligomers in neuroblastoma cell culture. The inventors further discretized the key molecular determinants responsible for the interaction of PSM$\alpha$3 and the pathogenic $\alpha$S species by deriving a family of sequence-simplified peptides having such specific $\alpha$S-binding and aggregation-inhibiting properties. The inventors thus address the afore-mentioned needs in the therapy and diagnosis of $\alpha$-synucleinopathies by providing a new family of rationally designed inhibitors of $\alpha$-synudein aggregation (also simply referred to as 'inhibitors' herein) which allow for specifically targeting the pathogenic species of $\alpha$S for diagnostic and therapeutic purposes.

Besides their therapeutic applications, these inhibitors of $\alpha$S aggregation can be used in very selective diagnostic assays which allows for diagnosis of $\alpha$-synucleinopathies including PD at least at the level of the current clinical diagnosis on a routine basis, and are suitable also for diagnosing early stage $\alpha$-synudeinopathies. Thereby it becomes possible to reach a major goal in the PD field, which is enabling therapeutic interventions early on, before substantial brain damage has occurred. In particular, the inventors developed a novel optical peptide-based nanoplasmonic biosensor which targets a-Syn oligomers with an affinity in the picomolar range, equivalent to that of an antibody and without interference from soluble monomeric $\alpha$-Syn. Being based on optical biosensing, this biosensor allows for real-time monitoring of the recognition events under label-free conditions with outstanding sensitivity and reproducibility.

Accordingly, the present invention relates to *in-vitro* methods for detecting $\alpha$-synuclein aggregates in a biological sample and corresponding kits as well as to sensors of a plasmonic sensor device as defined in the claims which utilize a member of the afore-mentioned novel family of inhibitors of $\alpha$-synudein aggregation.

Further aspects of the present invention relate to these inhibitors of $\alpha$-synudein aggregation for use in medicine, in particular in the treatment and prophylaxis of a synucleinopathy in a mammalian subject as defined in the claims, and to corresponding methods for the treatment or the prophylaxis of a synucleinopathy in a mammalian subject as also defined in the claims.

The invention also relates to pharmaceutical compositions comprising an inhibitor of $\alpha$-synudein aggregation as defined in the claims.

In an additional aspect, the invention relates to inhibitors of $\alpha$-synudein aggregation as defined in the claims which are characterized by the key molecular determinants responsible for interaction with toxic $\alpha$-synudein species and inhibiting $\alpha$-synundein aggregation determined by the inventors.

## DETAILED DESCRIPTION OF THE PREFERRED EMBODIMENTS

The present disclosure provides a novel family of inhibitors of $\alpha$-synudein aggregation, their use in therapeutic, prophylactic and diagnostic methods as well as related subject matter including pharmaceutical compositions, sensors for plasmonic sensor devices and kits.

Inhibitors of $\alpha$-synuclein aggregation

The inhibitors of $\alpha$-synudein aggregation of the present disclosure (also simply referred to as 'inhibitors' herein) are compounds comprising a peptide having specific structural and physicochemical features which allow for the inhibitors to specifically bind to pathogenic $\alpha$-synudein species and inhibit $\alpha$-synudein aggregation.

Specifically, the peptide has:

- an amphipathic $\alpha$-helical structure, and
- a net charge that is 0 or positive (meaning that the peptide is neutral or cationic) at pH 7.4.

The $\alpha$-helical propensity of a peptide can be characterized from its amino acid sequence by the algorithm described in Muñoz et al., 1994 that is based on the helix-coil transition theory and yields a so-called AGADIR value, wherein higher AGADIR values correlate with higher $\alpha$-helical propensity and values close to zero with lower $\alpha$-helical propensity. A web-based tool for calculating the AGADIR value of a given amino acid sequence is available in the internet from the Centre de Regulació Genòmica (http://agadir.crg.es). The term '$\alpha$-helical structure' as used herein refers to an AGADIR value of 1 or greater using the default settings (pH 7, Temperature 278 K, ionic strength 0.1). For example, the AGADIR value can be 2 or greater, or can be in the range of 1-90, or 2-80.

The term 'amphipathic' is generally used in the art to identify compounds which possess both hydrophilic and lipophilic properties (in the same compound). Common amphipathic compounds include - to name only a few - detergents such as sodium dodecyl sulfate (SDS), phospholipids, cholesterol, glycolipids, fatty acids and saponins. In the case of the helical peptides - such as the $\alpha$-helical peptides comprised by the inhibitors of the present disclosure - the amphipathic character results from the specific spatial distribution of hydrophilic and lipophilic amino acid residues which results in the helix having a hydrophilic face and an (opposite) lipophilic face. The amphipathic character (i.e. the degree of the amphiphilicity) of a helical peptide can be quantified based on its amino acid sequence by the algorithm described in Eisenberg et al., 1982 which calculates a mean hydrophobic moment (herein also referred to as 'helical hydrophobic moment'), wherein a higher helical hydrophobic moment means that the peptide has a more pronounced amphipathic character perpendicular to its axis. A web-based tool (HeliQuest) to screen peptide sequences with specific $\alpha$-helical properties, including their helical hydrophobic moment, is described in Gautier et al., 2008, and is available in the internet (https://heliquest.ipmc.cnrs.fr). Helical hydrophobic moment is calculated using the following settings: Helix type ($\alpha$), window size (Full). The term 'amphipathic' as used herein refers to a helical hydrophobic moment of 0.1 or greater such as, e.g., 0.2 or greater, or 0.3 or greater. Particularly, the helical hydrophobic moment can be in the range of 0.10-0.90, 0.15-0.80, 0.20-0.70, or 0.25-0.60.

At the physiological pH of 7.4, the amphipathic $\alpha$-helical peptide comprised by the inhibitors of the present disclosure have a net charge of 0 (i.e., are not charged) or a positive net charge (i.e. are cationic). Without wishing to be bound by theory, it is believed that this facilitates binding to the relevant anionic structures found in in toxic $\alpha$-synudein

oligomers and α-synudein fibrils. The net charge of a peptide can be calculated using a web-based tool (PROTEIN CALCULATOR v3.4) freely available on the internet (http://protcalc.sourceforge.net/) by introducing the peptide sequence, checking the option "Charge at pH" and selecting pH 7.4. The net charge at pH 7.4 of the amphipathic α-helical peptide of the present disclosure is 0 or greater such as, e.g., 0.1 or greater, or 0.5 or greater, or +1 or greater. Particularly the net charge can be in the range of from 0 to +10, from 0 to +7, from 0 to +6, from 0.1 to +5, from 0.1 to +4, from 0.5 to +3, or from 0.5 to +2.5.

The amphipathic α-helical peptide comprised by the inhibitors of the present disclosure is a peptide of at least 10 consecutive amino acid residues, for example a peptide of at least 12, at least 15, at least 19, such as 10-100, 12-60, 15-50 or 19-40 consecutive amino acid residues.

The term 'peptide' is used herein with its commonly accepted meaning in the art. Due to their length of at least 10 amino acid residues, the amphipathic α-helical peptides of the present disclosure are polypeptides.

The term 'amino acid' is used herein with its commonly accepted meaning in the art of biochemistry. The term 'amino acid residue' - according to is commonly accepted meaning in the art - and refers to the monomeric units which are linked to each other by peptidic bonds along a peptide chain. The amino acids residues comprised by the amphipathic α-helical peptides of the present disclosure can be residues of proteinogenic amino acids or residues of non-proteinogenic amino acids. include, but not limited to, proteinogenic amino acids and non-proteinogenic amino acids. Proteinogenic amino acids are α-amino acids and include canonical amino acids (arginine, histidine, lysine, aspartic acid, glutamic acid, serine, threonine, asparagine, glutamine, cysteine, glycine, proline, alanine, isoleucine, leucine, methionine, phenylalanine, tryptophan, tyrosine and valine) and non-canonical amino acids (e.g., selenocysteine, pyrrolysine), in particular the L-stereoisomers thereof. Examples of non-proteinogenic amino acids include ornithine, 3-aminopropanoic acid, homoarginine, citrulline, homocitrulline, homoserine, γ-aminobutyric acid, sarcosine, 2-aminoadipic acid, homocysteine, β-alanine, β-aminoisobutyric acid, β-leucine, β-lysine, β-arginine, β- tyrosine, β-phenylalanine, isoserine, β-glutamic acid, β-tyrosine, β-dopa (3,4-dihydroxy-L- phenylalanine), 2-aminoisobutyric acid, isovaline, di-n-ethylglycine, N-methyl-alanine, 4-hydroxyproline, 5-hydroxylysine, 3-hydroxyleucine, 4-hydroxyisoleucine, 5-hydroxy-L-tryptophan, 1-aminocyclopropyl-1-carboxylic acid and azetidine-2-carboxylic acid. Preferably, the amino acid residues within the amphipathic α-helical peptides are α-amino acids which are linked via α-peptide bonds.

In specific embodiments, the present disclosure relates to an inhibitor of α-synudein aggregation comprising an amphipathic α-helical peptide as described herein with the proviso that this inhibitor is not selected from PSMα3, LL-37 and β-synuclein. The amino acid sequences of PSMα3, LL-37 and β-synuclein are set forth in SEQ ID NO:2, SEQ ID NO:8 and SEQ ID NO:9, respectively.

Preferably, the inhibitors of the present disclosure are capable of binding to toxic α-synuclein oligomers and α-synudein fibrils with higher affinity than to monomeric α-synuclein. This binding preference for toxic α-synudein oligomers and α-synudein fibrils over monomeric α-synudein can be determined using techniques for analyzing the binding between chemical entities which are generally known in the art such as, for example, ELISA, Surface Plasmon Resonance (SPR) spectroscopy, dual-colour fluorescence cross-correlation spectroscopy (dcFCCS), single-molecule Förster resonance energy transfer (smFRET), and total internal reflection fluorescence microscopy (TIRFM) (Tokuda et al., 2010, Honarmand et al., 2019, Sun et al., 2017, Bacia et al., 2007, Nath et al., 2010, Horrocks et al., 2016). In particular, the higher affinity of the inhibitor to toxic α-synudein oligomers and α-synudein fibrils than to monomeric α-synudein can be determined by dual-colour fluorescence cross-correlation spectroscopy (dcFCCS) as described in example 9 or by single-molecule Förster resonance energy transfer (smFRET) as described in example 10. Preferably, the thereby determined binding intensity (relative binding affinity) of the inhibitor to toxic α-synudein oligomers and α-synudein fibrils is at least 2 times higher than to monomeric α-synudein.

The monomeric α-synuclein, toxic α-synudein oligomers and α-synudein fibrils used in these analyses can be prepared by methods known in the art (Pujols et al., 2018, Chen et al., 2018) or as described herein in example 1 (monomeric α-synudein), example 2 (labeled α-synudein), example 3 (toxic α-synudein oligomers) and example 5 (a-synuclein fibrils).

Typically, the inhibitor is capable of inhibiting in vitro α-synudein aggregation at equimolar concentrations of α-synudein and inhibitor or, preferably, at a molar excess of α-synuclein, for example at a 2-fold, 4-fold, 6-fold, 8-fold, 10-fold or 15-fold molar excess of α-synuclein.

The inhibition of in vitro α-synudein aggregation can be determined, for example, after contacting the inhibitor with α-synuclein, particularly α-synudein monomers (e.g., as prepared in example 1), for 32 h under suitable conditions, i.e. at conditions where, in the absence of the inhibitor, detectable amounts of α-synudein aggregates are formed.

A suitable marker for monitoring α-synudein aggregation by fluorescence measurements is thioflavin-T which can be measured, e.g., at about 450 nm excitation and about 480-510 nm emission. Without wishing to be bound by theory it is believed that the thioflavin-T binds to ordered structures such as found in α-synuclein fibril wherein said binding results in a red shift of the excitation wave length from 336 nm (of free dissolved thioflavin-T) to 450 nm (of bound thioflavin-T). Inhibition of α-synudein aggregation is thus detectable by a reduction (quenching) of the thioflavin-T fluorescence obtained at about 450 nm (e.g., 430-450 nm) excitation. For example, the inhibition of in vitro α-synudein aggregation can be determined by an $\alpha$-synudein aggregation kinetics assay as described in example 6.

The inhibitors of the present disclosure are typically capable of inhibiting the deleterious effects of pathogenic $\alpha$-synudein species, particularly toxic $\alpha$-synudein oligomers or $\alpha$-synudein fibrils, in cells. Toxic oligomers and $\alpha$-synudein fibrils are $\alpha$-synuclein aggregates which are distinguished from non-toxic aggregates (non-toxic $\alpha$-synudein oligomers) by their deleterious effects on cells.

In the context of synucleinopathies, these cells are typically neuronal cells. These deleterious effects can include an increase in the level of intracellular oxygen species and/or membrane disruption (Fusco et al., 2017).

The ability of the inhibitors to inhibit the deleterious effects of pathogenic $\alpha$-synudein species can thus be determined as ability to inhibit, *in vitro,* an increase in the level of intracellular reactive oxygen species in neuronal cells, wherein said increase is induced by treating the cells with toxic $\alpha$-synudein oligomers. In corresponding *in vitro* assays for determining said inhibition the neuronal cells can be, for example, human SH-SY5Y neuroblastoma cells such as the cells available from ATCC, #CRL-2266 or DSMZ #ACC 209. For example, the inhibition of the toxic $\alpha$-synudein oligomer induced increase in the level of intracellular reactive oxygen species can be determined by an assay as described in example 7.

The ability of the inhibitors to inhibit the deleterious effects of pathogenic $\alpha$-synudein species can also be determined as ability to inhibit, *in vitro,* the cell membrane disruption induced by toxic $\alpha$-synudein oligomers. Specifically, such *in vitro* assays examine the cell membrane disruption in neuronal cells such as, for example, human SH-SY5Y neuroblastoma cells as described herein. For example, the inhibition of the toxic $\alpha$-synudein oligomer induced cell membrane disruption can be determined by an assay as described in example 8.

Particular examples of inhibitors of $\alpha$-synudein aggregation according to the present disclosure are PSM$\alpha$3 (SEQ ID NO:2), All-Leu (SEQ ID NO:4), All-Leu-19 (SEQ ID NO:5), scaffold-19 (SEQ ID NO:6), and LL-37 (SEQ ID NO:8). Further inhibitors of the present disclosure include those comprising an amino acid sequence having at least 84%, at least 88, at least 90%, at least 94%, or at least 97%, or 100% sequence identity to an amino acid sequence selected from SEQ ID NOs:2, 4, 5, 6 and 8.

Inhibitors of the present disclosure can be obtained by screening a plurality of amino acid sequences for peptides:

(i) comprising at least 10 consecutive amino acid residues, for example a peptide of at least 12, at least 15, at least 19, such as 10-100, 12-60, 15-50 or 19-40 consecutive amino acid residues, and

(ii) having a net charge at pH 7.4 that is 0 or positive, for example a net charge that is + 0.1 or greater, +0.5 or greater, or +1 or greater, or is in the range of from 0 to +10, from 0 to +7, from 0 to +6, from 0.1 to +5, from 0.1 to +4, from 0.5 to +3, or from 0.5 to +2.5, and

(iii) AGADIR value of 1 or greater, and

(iv) a helical hydrophobic moment of 0.1 or greater, for example 0.2 or greater, or 0.3 or greater, or is in the range of 0.10-0.90, 0.15-0.80, 0.20-0.70, or 0.25-0.60.

Said plurality of amino acid sequences can be amino acid sequences within an existing peptide database such as, for example, the EROP-Moscow oligopeptide database which comprises peptides of up to about 50 amino acid residues and is described in Zamyatnin et al., 2006.

The inhibitors of the present disclosure, and in particular the amphipathic $\alpha$-helical peptide thereof, can be prepared by chemical synthesis or recombinant expression or a combination of both, typically followed by isolation / purification steps. Methods for peptide synthesis are well known in the art and include, for example, solid-phase peptide synthesis (also known as Merrifield synthesis) wherein a peptide chain is assembled by successive coupling of (activated) amino acids to the thereby growing peptide chain that is immobilized on an insoluble support. Commonly used reagents for amide bond formation include carbodiimides, ammonium/uronium reagents (e.g., HATU, HBTU/TBTU, HCTU), phosphonium reagents (e.g., PyBOP and PyAOP), and propanephosphonic acid anhydride. To avoid undesirable side reactions such as self-coupling (polymerization) of the activated amino acid, the N-terminal and side chain amino groups are usually protected with suitable protecting groups. Common examples of such amine-protecting groups include Boc and Fmoc.

Methods for the recombinant expression of peptides are well known in the art. Generally, a polynucleotide encoding the to be expressed peptide - expediently in an expression construct - is introduced into a host organism or cell (e.g., a bacterium or yeast cell) which then is cultured under conditions which allow for the expression of said peptide.

The amphipathic $\alpha$-helical peptides of the present disclosure can be used as such as can be coupled to one or more than one peptidic or non-peptidic molecule such as, for example, a detectable label.

Labeling strategies for polypeptides known in the art can be used for the inhibitors of the present disclosure including, for example, covalent attachment to labeling molecules such as biotin, reporter enzymes (e.g., alkaline phosphatase, horseradish peroxidase, luciferase), and fluorophores (e.g., fluorescein, fluorophores of the Alexa Fluor dye family (such as Alexa Fluor 488, 546, 633, etc., Invitrogen, Carlsbad, CA, USA), Oregon Green 488 (Invitrogen, Carlsbad, CA, USA)), and incorporation of (or covalent coupling with molecules comprising) one or more radioactive isotopes (e.g., $^{14}C$, $^{3}H$, $^{35}S$, $^{32}P$, $^{131}I$, $^{125}I$).

Medical use

The inhibitors of α-synudein aggregation according to the present disclosure can be used in medicine. In particular, the inhibitors can be used in the treatment and prophylaxis of a synucleinopathy in a mammalian subject. Accordingly, the present disclosure comprises the use of such inhibitor in medicine, the use of such inhibitor in the treatment and prophylaxis of a synucleinopathy in a mammalian subject, as well as a method for the treatment and prophylaxis of a synucleinopathy in a mammalian subject wherein a pharmaceutically effective amount of an inhibitor of α-synudein aggregation is administered to the subject.

Synucleinopathies are a group of disorders characterized by protein deposition in inclusions located in neuronal and/or glial cells. Said protein deposits are referred to as Lewy bodies and Lewy neurites. The major component of said protein deposits is α-synudein. The α-synudein aggregation observed in these disorders is believed to be responsible for the neurotoxicity underlying their pathology. Various animal models have been developed to study the formation α-synuclein-containing protein deposits and their pathology in synucleinopathies. See, e.g., Benskey et al., 2016 and the references cited therein.

Administration of an inhibitor of the present disclosure, can prevent and/or delay the onset or the progression of the formation of α-synudein deposits in a mammalian subject, e.g. a mammal known or suspected to have or being at risk of developing a synucleinopathy.

The treatment of a synucleinopathy as described herein can comprise one or more of the following: reducing or ameliorating the severity and/or duration of the synucleinopathy or one or more symptoms thereof, preventing the advancement of the synucleinopathy, causing regression of the synucleinopathy, preventing or delaying the recurrence, development, onset or progression of the synucleinopathy or one or more symptoms thereof, enhancing or improving the therapeutic effect(s) of another therapy (e.g., another therapeutic drug) against the synucleinopathy. Unless indicated otherwise, a treatment of a synucleinopathy as described herein may be a prophylactic treatment, e.g. in a subject at risk of developing a synucleinopathy. Prophylaxis or a prophylactic treatment of a synucleinopathy as described herein can include one or more of the following: preventing or delaying the onset of the synucleinopathy or one or more symptoms thereof, enhancing or improving the prophylactic effect of another therapy (e.g., another prophylactic drug) against the synucleinopathy.

The mammalian subject of the treatment or the prophylaxis can be, for example a mouse, cat, dog, monkey or human, and is preferably a human. The subject is expediently an individual known or suspected to suffer from a synucleinopathy, or at risk of developing a synucleinopathy.

Whether treatment or prophylaxis of a synucleinopathy using an inhibitor of the present disclosure is indicated, and in which form it is to take place, depends on the individual case and is subject to medical assessment (diagnosis) which takes into consideration signs, symptoms and/or malfunctions which are present, the risks of developing particular signs, symptoms and/or malfunctions, and other factors.

As a rule, treatment or prophylaxis is effected by means of single or repeated administration of a pharmaceutically effective amount of the inhibitor, where appropriate together, or alternating, with other drugs or drug-containing compositions. As used herein, the term "pharmaceutically effective amount" refers to the amount of a therapy which is sufficient to achieve one or more of the following: reduce or ameliorate the severity and/or duration of the disease or one or more symptoms thereof, prevent the advancement of the disease, cause regression of the disease, prevent or delay the recurrence, development, onset or progression of the disease or one or more symptoms thereof, enhance or improve the therapeutic effect(s) of another therapy or prophylaxis (e.g., another therapeutic or prophylactic drug) against the disease.

Examples of synucleinopathies which can be treated, delayed or prevented as described herein include Parkinson's Disease, Dementia with Lewy Bodies, Multiple System Atrophy, Pure Autonomic Failure, Lewy Body Variant of Alzheimer's Disease and Neurodegeneration with Brain Iron Accumulation. In preferred aspects, the synucleinopathy to be treated, delayed or prevented as described herein is Parkinson's Disease.

In Parkinson's Disease, Dementia with Lewy Bodies, Pure Autonomic Failure and Lewy Body Variant of Alzheimer's Disease, the α-synuclein-containing protein deposits are primarily detected in neurons. In Multiple System Atrophy, the deposits are primarily in glial cells. In Neurodegeneration with Brain Iron Accumulation α-synuclein-containing protein deposits are detected in both neurons and glial cells.

Parkinson's Disease is a progressive disease which usually manifests after the age of 50 years, although early-onset cases (before 50 years) are known. The majority of the cases are sporadic suggesting a multifactorial etiology based on environmental and genetic factors. However, in some cases, there is a positive family history for the disease.

Such familial forms of the Parkinson's Disease usually begin at an earlier age. See Tsigelny et al., 2015. Certain point mutations of human α-synudein are known in the art to significantly increase oligomerization. For example, the point mutations A30P, E46K, H50Q, G51D, A53E, A53F and A53T of α-synudein are known to cause familial forms of Parkinson's Disease. In particular aspects, the synucleinopathy is thus a familial synucleinopathy such as, e.g., familiar Parkinson's Disease, and/or the subject of the treatment or the prophylaxis presents multiplications of the gene encoding α-synudein (i.e., the SNCA gene, NCBI Gene ID: 6622), or expresses α-synudein comprising at least one amino acid substitution selected from a proline at position 30, a lysine at position 46, a glutamine at position 50, an aspartate at position 51, and a threonine, a valine or a glutamate at position 53, wherein the numbering of said amino acid positions is relative to the of full length α-synudein as set forth in SEQ ID NO:1.

In view of their *in vitro* activity, particular medical uses of the inhibitors of the present disclosure include the use in inhibiting α-synudein aggregation in the subject, specifically to reduce α-synuclein-induced damage to cells (particularly neuronal cells) in the subject. Such reduction of the α-synuclein-induced cellular damage can mean a reduction in the level of intracellular reactive oxygen species.

The inhibitors of the present disclosure, can be administered in the form of a pharmaceutical composition that comprises one or more of said inhibitors and at least one pharmaceutically acceptable carrier. The composition may optionally further comprise one or more other therapeutic or prophylactic drugs for treating a synucleinopathy.

The term "pharmaceutically acceptable", as used herein, refers to a compound that does not cause acute toxicity when administered in an amount that is required for medical treatment or medical prophylaxis. Expediently, all components of the pharmaceutical composition of the present disclosure are pharmaceutically acceptable.

A carrier can be a solid, semisolid or liquid material which serves as vehicle or medium for the pharmaceutically active compound. Pharmaceutically acceptable carriers are known in the art and are chosen according to the dosage form and the desired way of administration. For example, the composition can be formulated for oral, rectal, transdermal, subcutaneous, intravenous, intramuscular or intranasal administration.

The pharmaceutical compositions of the present disclosure can be, for example, solid dosage forms, such as powders, granules, tablets, in particular film tablets, lozenges, sachets, cachets, sugar-coated tablets, capsules, such as hard gelatin capsules and soft gelatin capsules, suppositories or vaginal medicinal forms, semisolid medicinal forms, such as ointments, creams, hydrogels, pastes or plasters, and also liquid medicinal forms, such as solutions, emulsions, in particular oil-in-water emulsions, suspensions, for example lotions, injection preparations and infusion preparations, and eyedrops and eardrops. Implanted release devices can also be used for administering inhibitors of the present disclosure. In addition, it is also possible to use liposomes or microspheres.

Suitable carriers are listed in the specialist medicinal monographs. In addition, the compositions can comprise pharmaceutically acceptable auxiliary substances, such as wetting agents; emulsifying and suspending agents; preservatives; antioxidants; anti-irritants; chelating agents; coating auxiliaries; emulsion stabilizers; film formers; gel formers; odor masking agents; taste corrigents; resin; hydrocolloids; solvents; solubilizers; neutralizing agents; diffusion accelerators; pigments; quaternary ammonium compounds; refatting and overfatting agents; raw materials for ointments, creams or oils; silicone derivatives; spreading auxiliaries; stabilizers; sterilants; suppository bases; tablet auxiliaries, such as binders, fillers, glidants, disintegrants or coatings; propellants; drying agents; opacifiers; thickeners; waxes; plasticizers and white mineral oils. Such auxiliary substances are also well known in the art.

The formulation of the composition is expediently chosen according to the intended way of administration. Suitable formulation types for the different ways of administration are known in the art and described herein.

Diagnostic use

The ability of inhibitors of the present disclosure to bind pathogenic α-synudein species specifically (particularly compared to non-pathogenic α-synudein species such as α-synudein monomers and non-toxic α-synudein oligomers) and with high binding affinity - meaning $K_D$ values equivalent to those of antibodies which are typically in the low nanomolar range - are also particularly advantageous for their diagnostic use.

The present disclosure thus provides *in-vitro* methods for detecting α-synudein aggregates in a biological sample of a mammalian subject. The detection methods comprise contacting a herein-described inhibitor of α-synudein aggregation with the sample and then detecting the binding of α-synudein aggregates comprised in the sample to the inhibitor.

Said detection methods can be used in the diagnosis of synucleinopathy. Accordingly, the detection method of the present disclosure can be a method of diagnosing a synucleinopathy in the mammalian subject, wherein the detected α-synudein aggregate level being higher than the α-synudein aggregate levels in healthy subjects of the same species indicates that the mammalian subject suffers from a synucleinopathy, for example one of the specific synucleinopathies described herein-above.

The mammalian subject can be, for example a mouse, cat, dog, monkey or human, and is preferably a human. The biological sample of the mammalian subject is expediently a sample that can reasonably be expected to comprise pathogenic α-synuclein species, if such were present in the mammalian subject. Preferably, the biological sample is a sample of the cerebrospinal fluid of the subject.

The binding of α-synudein aggregates comprised in the biological sample to the inhibitor can be determined using techniques for analyzing the binding between chemical entities which are generally known in the art and includes techniques such as, for example, ELISA (Tokuda et al., 2010; Weng et al., 2015), fluorescence cross-correlation spectroscopy (FCCS) and TIRFM, wherein the detecting agent (which here is the inhibitor) detectably labeled, e.g. which a label as described herein-above. Alternatively - and preferably - label-free techniques are used, particularly those wherein the inhibitor is immobilized. Label-free techniques for detecting the interaction of biomolecules are well known in the art and include in particular techniques using SPR sensors (SPR spectroscopy) and photonic sensors such as, for example, those described by Nguyen et al., 2015 and Singh et al., 2006.

Such sensors are also called 'biosensors' when used for detecting the interaction of biomolecules including polypeptides, and are particularly advantageous as they highly sensitive and allow for high-throughput and miniaturized platforms. Specifically, such biosensor can be integrated in a lab-on-a-chip platform that allows the fabrication of smaller, cheaper and easy-to-use devices suitable for point-of-care biomarker analysis.

In a particular detection method (or diagnostic method) of the present disclosure, the inhibitor can be immobilized on the sensor surface of a plasmonic sensor device. In this case, binding of α-synudein aggregates to the immobilized inhibitor can be detected by Surface Plasmon Resonance (SPR) spectroscopy, preferably by Localized Surface Plasmon Resonance (LSPR) spectroscopy. SPR including LSPR are techniques well known in art (Nguyen et al., 2015; Singh et al., 2006; Csáki et al., 2018).

The inhibitor can be immobilized on the surface of the sensor by well-established methods which are usually also described in the supplier's manual for the sensor. Such methods include covalent coupling using functional groups (e.g., free amine, thiol, hydroxyl and/or carboxyl groups) of the inhibitor and affinity capturing systems. For covalent coupling the inhibitor can, for example, be treated with N-hydroxysuccinimide (NHS) and 3-(Ethyliminomethyleneamino)-N,N-dimethylpropan-1-amine (EDC) followed by 2-(2-pyridinyldithio)ethaneamine (PDEA) so as to introduce a reactive disulphide group that can react with a thiol group located on the sensor surface. Alternatively, the inhibitor can be treated with NHS/EDC followed by cysteamine and a thiol compound such as dithioerythritol (DTE) or dithiothreitol (DTT) so as to introduce a thiol group that can react with a reactive disulphide group located on the sensor surface. Inhibitors comprising cis-diols can be immobilized by oxidizing them (e.g. with sodium metaperiodate) to an aldehyde group which can react with a hydrazine group located on the sensor surface. The formed hydrazine bond is then stabilized by reduction (e.g., with cyanoborhydride).

In immobilization via affinity capturing systems, inhibitor comprises (or is beforehand coupled to) a covalently linked tag which is then captured by a corresponding high-affinity binding partner located on the sensor surface. In a preceding step, said high-affinity binding partner can immobilized on the sensor surface by covalent coupling via functional groups (e.g., free amine, thiol, hydroxyl and/or carboxyl groups) as described above. Particular examples of tag-binding partner pairs include hexahistidine tag / nitrilotriacetic acid (NTA) or tris-NTA; streptavidin / biotin, GST / anti-GST antibody; myc / anti-myc antibody; MBP / anti-MBP antibody; and FLAG / anti-FLAG antibody - wherein, in principle, either the tag or its binding partner can be present in (or beforehand coupled to) the inhibitor, while the corresponding other of the pair (i.e., binding partner or tag) is immobilized (already prior to capturing the inhibitor) on the sensor surface.

Useful negative controls for the detection method of the present disclosure include biological samples of the same type from healthy subjects, such as, for example, the cerebrospinal fluid of one or more healthy persons as negative control for the cerebrospinal fluid of a human subject to be tested for α-synudein aggregates.

The present disclosure further refers to a sensor of a plasmonic sensor device, the sensor carrying on its surface an immobilized inhibitor of α-synudein aggregation as described herein. For example, at least part of the surface of the sensor can be formed by nanoparticles. Preferably, the plasmonic sensor device is a Localized Surface Plasmon Resonance (LSPR) spectrometer.

The present disclosure also refers to kits for detecting α-synudein aggregates in a biological sample. Such kits comprise an inhibitor of α-synudein aggregation as described herein and a control sample comprising α-synudein aggregates. For example, the control sample comprises α-synudein fibrils which are obtainable as described in example 5 herein.

The disclosure further comprises the following embodiments E1-E68:

E1. An inhibitor of α-synudein aggregation for use in the treatment and prophylaxis of a synucleinopathy in a mammalian subject, preferably a human, wherein the inhibitor comprises a peptide of at least 10 consecutive amino acid residues, said peptide having:

- an amphipathic α-helical structure as defined by an AGADIR value of 1 or greater and by a helical hydrophobic moment of 0.1 or greater, and

- a net charge that is 0 or positive at pH 7.4.

E2. The inhibitor for use according to E1, wherein the synucleinopathy is selected from Parkinson's Disease, Dementia with Lewy Bodies, Multiple System Atrophy, Pure Autonomic Failure, Lewy Body Variant of Alzheimer's Disease and Neurodegeneration with Brain Iron Accumulation.

E3. The inhibitor for use according to E1 or E2, wherein the synucleinopathy is familial.

E4. The inhibitor for use according to any one of E1-E3, wherein the subject of the treatment or the prophylaxis presents multiplications of the gene encoding $\alpha$-synuclein (*SNCA* gene), or expresses $\alpha$-synudein comprising at least one amino acid substitution selected from:

(i) a proline at position 30,

(ii) a lysine at position 46,

(iii) a glutamine at position 50,

(iv) an aspartate at position 51, and

(v) a threonine, a valine or a glutamate at position 53;

the numbering of said amino acid positions being relative to the of full length $\alpha$-synuclein as set forth in SEQ ID NO:1.

E5. The inhibitor for use according to any one of E1-E4, wherein the inhibitor is for use in delaying the onset or the progression of the synucleinopathy.

E6. The inhibitor for use according to any one of E1-E5, wherein the amphipathic $\alpha$-helical peptide is a peptide of at least 12, at least 15, at least 19, such as 10-100, 12-60, 15-50 or 19-40 consecutive amino acid residues.

E7. The inhibitor for use according to any one of E1-E6, wherein the net charge of the amphipathic $\alpha$-helical peptide at pH 7.4 is + 0.1 or greater, +0.5 or greater, or +1 or greater, or is in the range of from 0 to +10, from 0 to +7, from 0 to +6, from 0.1 to +5, from 0.1 to +4, from 0.5 to +3, or from 0.5 to +2.5.

E8. The inhibitor for use according to any one of E1-E7, wherein the helical hydrophobic moment of the amphipathic $\alpha$-helical peptide is 0.2 or greater, or 0.3 or greater, or is in the range of 0.10-0.90, 0.15-0.80, 0.20-0.70, or 0.25-0.60.

E9. The inhibitor for use according to any one of E1-E8, wherein the inhibitor is capable of binding to toxic $\alpha$-synudein oligomers and $\alpha$-synudein fibrils with higher affinity than to monomeric $\alpha$-synudein.

E10. The inhibitor for use according to E9, wherein the higher affinity of the inhibitor to toxic $\alpha$-synudein oligomers and $\alpha$-synudein fibrils than to monomeric $\alpha$-synuclein can be determined by dual-colour fluorescence cross-correlation spectroscopy (dcFCCS) as described in example 9 or by single-molecule Förster resonance energy transfer (smFRET) as described in example 10.

E11. The inhibitor for use according to E10, wherein the inhibitor is capable of binding to toxic $\alpha$-synudein oligomers and $\alpha$-synudein fibrils with at least 2-times higher affinity than to monomeric $\alpha$-synudein.

E12. The inhibitor for use according to any one of E9-E11, wherein the toxic $\alpha$-synudein oligomers are obtainable by the method described in example 4, and the $\alpha$-synudein fibrils are obtainable by the method described in example 5.

E13. The inhibitor for use according to any one of E1-E12, wherein the inhibitor is capable of inhibiting *in vitro* $\alpha$-synudein aggregation at equimolar concentrations of $\alpha$-synudein and inhibitor.

E14. The inhibitor for use according to E13, wherein the inhibitor is capable of inhibiting *in vitro* $\alpha$-synudein aggregation at 2-fold, 4-fold, 6-fold, 8-fold, 10-fold or 15-fold molar excess of $\alpha$-synudein.

E15. The inhibitor for use according to E13 or E14, wherein the inhibition of *in vitro* $\alpha$-synudein aggregation can be determined after contacting the inhibitor with α-synuclein for 32 h under suitable conditions.

E16. The inhibitor for use according to any one of E13-E15, wherein the inhibition of *in vitro* α-synudein aggregation can be determined using thioflavin-T.

E17. The inhibitor for use according to any one of E13-E16, wherein the inhibition of *in vitro* α-synudein aggregation can be determined by an α-synudein aggregation kinetics assay as described in example 6.

E18. The inhibitor for use according to any one of E1-E17, wherein the inhibitor is capable of inhibiting, *in vitro,* an increase in the level of intracellular reactive oxygen species in neuronal cells, wherein said increase is induced by treating the cells with toxic α-synudein oligomers.

E19. The inhibitor for use according to E18, wherein the neuronal cells are human SH-SY5Y neuroblastoma cells.

E20. The inhibitor for use according to E18 or E19, wherein the inhibition of the toxic α-synuclein oligomer induced increase in the level of intracellular reactive oxygen species is determined by an assay as described in example 7.

E21. The inhibitor for use according to any one of E1-E20, wherein the inhibitor is capable of inhibiting, *in vitro,* the cell membrane disruption induced by toxic α-synuclein oligomers.

E22. The inhibitor for use according to E21, wherein the inhibition of the toxic α-synuclein oligomer induced cell membrane disruption is determined by an assay as described in example 8.

E23. The inhibitor for use according to any one of E1-E22, wherein the inhibitor comprises an amino acid sequence having at least 84%, at least 88, at least 90%, at least 94%, or at least 97%, or 100% sequence identity to an amino acid sequence selected from SEQ ID NOs:2, 4, 5, 6 and 8.

E24. An *in-vitro* method for detecting α-synudein aggregates in a biological sample of a mammalian subject, wherein the method comprises contacting an inhibitor of α-synudein aggregation with the sample and then detecting the binding of α-synuclein aggregates comprised in the sample to the inhibitor, wherein the inhibitor comprises a peptide of at least 10 consecutive amino acid residues, said peptide having:

- an amphipathic α-helical structure as defined by an AGADIR value of 1 or greater and by a helical hydrophobic moment of 0.1 or greater, and

- a net charge that is 0 or positive at pH 7.4.

E25. The method of E24, wherein binding of α-synudein aggregates to the immobilized inhibitor is detected by a label-free technique for detecting the interaction of biomolecules.

E26. The method of E24 or E25, wherein the inhibitor is immobilized on the sensor surface of a plasmonic sensor device, and binding of α-synudein aggregates to the immobilized inhibitor is detected by Surface Plasmon Resonance (SPR) spectroscopy, preferably by Localized Surface Plasmon Resonance (LSPR) spectroscopy.

E27. The method of any one of E24-E26, wherein the amphipathic α-helical peptide is a peptide of at least 12, at least 15, at least 19, such as 10-100, 12-60, 15-50 or 19-40 consecutive amino acid residues.

E28. The method of any one of E24-E27, wherein the net charge of the amphipathic α-helical peptide at pH 7.4 is + 0.1 or greater, +0.5 or greater, or +1 or greater, or is in the range of from 0 to +10, from 0 to +7, from 0 to +6, from 0.1 to +5, from 0.1 to +4, from 0.5 to +3, or from 0.5 to +2.5.

E29. The method of any one of E24-E28, wherein the helical hydrophobic moment of the amphipathic α-helical peptide is 0.2 or greater, or 0.3 or greater, or is in the range of 0.10-0.90, 0.15-0.80, 0.20-0.70, or 0.25-0.60.

E30. The method of any one of E24-E29, wherein the inhibitor is capable of binding to toxic α-synudein oligomers and α-synudein fibrils with higher affinity than to monomeric α-synudein.

E31. The method of E30, wherein the higher affinity of the inhibitor to toxic α-synuclein oligomers and α-synudein fibrils than to monomeric α-synudein can be determined by dual-colour fluorescence cross-correlation spectroscopy (dcFCCS) as described in example 9 or by single-molecule Förster resonance energy transfer (smFRET) as described in example 10.

E32. The method of E31, wherein the inhibitor is capable of binding to toxic α-synuclein oligomers and α-synudein fibrils with at least 2-times higher affinity than to monomeric α-synudein.

E33. The method of any one of E30-E32, wherein
the toxic α-synudein oligomers are obtainable by the method described in example 4, and the α-synudein fibrils are obtainable by the method described in example 5.

E34. The method of any one of E24-E33, wherein the inhibitor is capable of inhibiting *in vitro* α-synudein aggregation at equimolar concentrations of α-synudein and inhibitor.

E35. The method of E34, wherein the inhibitor is capable of inhibiting *in vitro* α-synuclein aggregation at 2-fold, 4-fold, 6-fold, 8-fold, 10-fold or 15-fold molar excess of α-synudein.

E36. The method of E34 or E35, wherein the inhibition of *in vitro* α-synudein aggregation can be determined after contacting the inhibitor with α-synudein for 32 h under suitable conditions.

E37. The method of any one of E34-E36, wherein the inhibition of *in vitro* α-synuclein aggregation can be determined using thioflavin-T.

E38. The method of any of E34-E37, wherein the inhibition of *in vitro* α-synudein aggregation can be determined by an α-synudein aggregation kinetics assay as described in example 6.

E39. The method of any one of E24-E38, wherein the inhibitor is capable of inhibiting, *in vitro,* an increase in the level of intracellular reactive oxygen species in neuronal cells, wherein said increase is induced by treating the cells with toxic α-synudein oligomers.

E40. The method of E39, wherein the neuronal cells are human SH-SY5Y neuroblastoma cells.

E41. The method of E39 or E40, wherein the inhibition of the toxic α-synudein oligomer induced increase in the level of intracellular reactive oxygen species is determined by an assay as described in example 7.

E42. The method of any one of E24-E41, wherein the inhibitor is capable of inhibiting, *in vitro,* the cell membrane disruption induced by toxic α-synudein oligomers.

E43. The inhibitor for use according to E42, wherein the inhibition of the toxic α-synuclein oligomer induced cell membrane disruption is determined by an assay as described in example 8.

E44. The method of any one of E24-E43, wherein the inhibitor comprises an amino acid sequence having at least 84%, at least 88, at least 90%, at least 94%, or at least 97%, or 100% sequence identity to an amino acid sequence selected from SEQ ID NOs:2, 4, 5, 6 and 8.

E45. The method of any one E24-E44 that is a method of diagnosing a synucleinopathy in the mammalian subject, wherein the detected α-synudein aggregate level being higher than the α-synudein aggregate levels in healthy subjects of the same species indicates that the mammalian subject suffers from a synucleinopathy.

E46. The method of E45, wherein the synucleinopathy is selected from Parkinson's Disease, Dementia with Lewy Bodies, Multiple System Atrophy, Pure Autonomic Failure, Lewy Body Variant of Alzheimer's Disease and Neurodegeneration with Brain Iron Accumulation, and preferably is Parkinson's Disease.

E47. The method of E45 or E46, wherein the synucleinopathy is familial.

E48. The method of any one of E45-E47, wherein the subject presents multiplications of the gene encoding α-synudein (*SNCA* gene), or expresses α-synuclein comprising at least one amino acid substitution selected from:

(vi) a proline at position 30,

(vii) a lysine at position 46,

(viii) a glutamine at position 50,

(ix) an aspartate at position 51, and

(x) a threonine, a valine or a glutamate at position 53;

the numbering of said amino acid positions being relative to the of full length α-synuclein as set forth in SEQ ID NO:1.

E49. The method of any one of E24-E48, wherein the subject is a human.

E50. The method of any one of E23-E49, wherein the biological sample is a sample of the cerebrospinal fluid of the subject.

E51. A sensor of a plasmonic sensor device, the sensor carrying on its surface an immobilized inhibitor of α-synudein aggregation, wherein the inhibitor comprises a peptide of at least 10 consecutive amino acid residues, said peptide having:

- an amphipathic α-helical structure as defined by an AGADIR value of 1 or greater and by a helical hydrophobic moment of 0.1 or greater, and

- a net charge that is 0 or positive at pH 7.4.

E52. The sensor of E51, wherein at least part of the surface of the sensor is formed by nanoparticles, and wherein the plasmonic sensor device is preferably a Localized Surface Plasmon Resonance (LSPR) spectrometer.

E53. The sensor of E51 or E52, wherein the inhibitor is as defined in E27-E44.

E54. A kit for detecting α-synudein aggregates in a biological sample, wherein the kit comprises an inhibitor of α-synudein aggregation and a control sample comprising α-synudein aggregates, wherein the inhibitor comprises a peptide of at least 10 consecutive amino acid residues, said peptide having:

- an amphipathic α-helical structure as defined by an AGADIR value of 1 or greater and by a helical hydrophobic moment of 0.1 or greater, and

- a net charge that is 0 or positive at pH 7.4.

E55. The kit of E54, wherein the inhibitor is immobilized on the sensor surface of plasmonic sensor device, wherein the plasmonic sensor device is preferably a Localized Surface Plasmon Resonance (LSPR) spectrometer.

E56. The kit of E54 or E55, wherein the inhibitor is as defined in E27-E44.

E57. An inhibitor of α-synudein aggregation comprising a peptide of at least 10 consecutive amino acid residues, said peptide having:

- an amphipathic α-helical structure characterized by an AGADIR value of 1 or greater and by a helical hydrophobic moment of 0.1 or greater, and

- a net charge that is 0 or positive at pH 7.4;

provided that the inhibitor is not a peptide having the amino acid sequence of SEQ ID NO:2 or SEQ ID NO:8 or SEQ ID NO:9.

E58. The inhibitor of α-synudein aggregation according to E57, wherein the inhibitor is as defined in any one of E6-E23.

E59. A pharmaceutical composition comprising an inhibitor of α-synudein aggregation and at least one pharmaceutically acceptable carrier, wherein the inhibitor comprises a peptide of at least 10 consecutive amino acid residues, said peptide having:

- an amphipathic α-helical structure characterized by an AGADIR value of 1 or greater and by a helical hydrophobic moment of 0.1 or greater, and
- a net charge that is 0 or positive at pH 7.4.

E60. The pharmaceutical composition according to E59, wherein the inhibitor is as defined in any one of E6-E23, E57 and E58.

E61. An inhibitor of α-synudein aggregation for use in medicine, wherein the inhibitor comprises a peptide of at least 10 consecutive amino acid residues, said peptide having:

- an amphipathic α-helical structure characterized by an AGADIR value of 1 or greater and by a helical hydrophobic moment of 0.1 or greater, and

- a net charge that is 0 or positive at pH 7.4.

E62. The inhibitor for use according to E61, wherein the inhibitor is as defined in any one of E6-E23.

E63. A method for the treatment or the prophylaxis of a synucleinopathy in a mammalian subject, preferably a human, wherein a pharmaceutically effective amount of an inhibitor of α-synudein aggregation is administered to the subject, wherein the inhibitor comprises a peptide of at least 10 consecutive amino acid residues, said peptide having:

- an amphipathic α-helical structure characterized by an AGADIR value of 1 or greater and by a helical hydrophobic moment of 0.1 or greater, and

- a net charge that is 0 or positive at pH 7.4.

E64. The method of E63, wherein the inhibitor is as defined in any one of E6-E23.

E65. The method of E63 or E64, wherein the synucleinopathy synucleinopathy is selected from Parkinson's Disease, Dementia with Lewy Bodies, Multiple System Atrophy, Pure Autonomic Failure, Lewy Body Variant of Alzheimer's Disease and Neurodegeneration with Brain Iron Accumulation.

E66. The method of any one of E61-E65, wherein the synucleinopathy is familial.

E67. The method of any one of E63-E66, wherein the subject of the treatment or the prophylaxis presents multiplications of the gene encoding α-synudein (*SNCA* gene), or expresses α-synudein comprising at least one amino acid substitution selected from:

(xi) a proline at position 30,

(xii) a lysine at position 46,

(xiii) a glutamine at position 50,

(xiv) an aspartate at position 51, and

(xv) a threonine, a valine or a glutamate at position 53;

the numbering of said amino acid positions being relative to the of full length α-synuclein as set forth in SEQ ID NO:1.

E68. The method of any one of E63-E67, wherein the method delays the onset or the progression of the synucleinopathy.

**DESCRIPTION OF FIGURES**

**Figure 1** shows the main molecular features of four different $\alpha$-synudein species: monomer, non-toxic oligomer ("Oligomer type A*", obtainable by the method of example 4), toxic oligomer ("Oligomer type B*", obtainable by the method of example 3), fibrils. Values with dagger (†) represent extrapolations based on the average number of monomers in each oligomer/fibril.

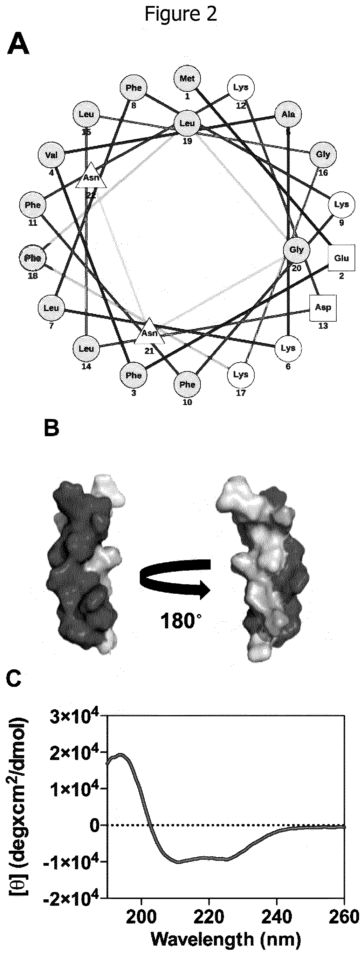

**Figure 2** illustrates the amphipathic character of PSM$\alpha$3. (A) Helical wheel projection of PMS$\alpha$3 sequence (non-polar residues: grey circles, polar basic residues; white circles, polar acid residues: squares, polar uncharged residues: triangles). (B) Three-dimensional structure of PSM$\alpha$3. (C) Circular dichroism (CD) spectrum of PSM$\alpha$3.

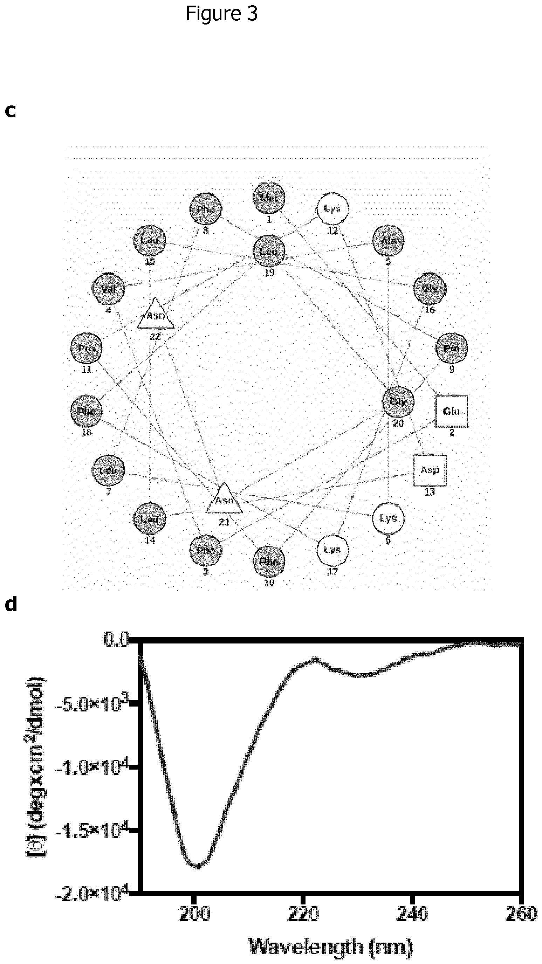

**Figure 3** shows the design of non-amphipathic PSM$\alpha$3 mutant dPSM$\alpha$3. (A) Computational proline scanning. Predicted $\alpha$-helical propensity represented with the AGADIR score; higher values correlate with higher $\alpha$-helical propensity and values close to zero with lower helical propensities. (B) Sequence alignment of PSM$\alpha$3 and dPSM$\alpha$3. (C) Helical wheel projection of dPSM$\alpha$3 showing the location of the introduced prolines. Non polar residues; grey circles, polar basic residues; white circles, polar acid residues; squares, polar uncharged residues; triangles. (D) Circular dichroism spectrum of dPSM$\alpha$3.

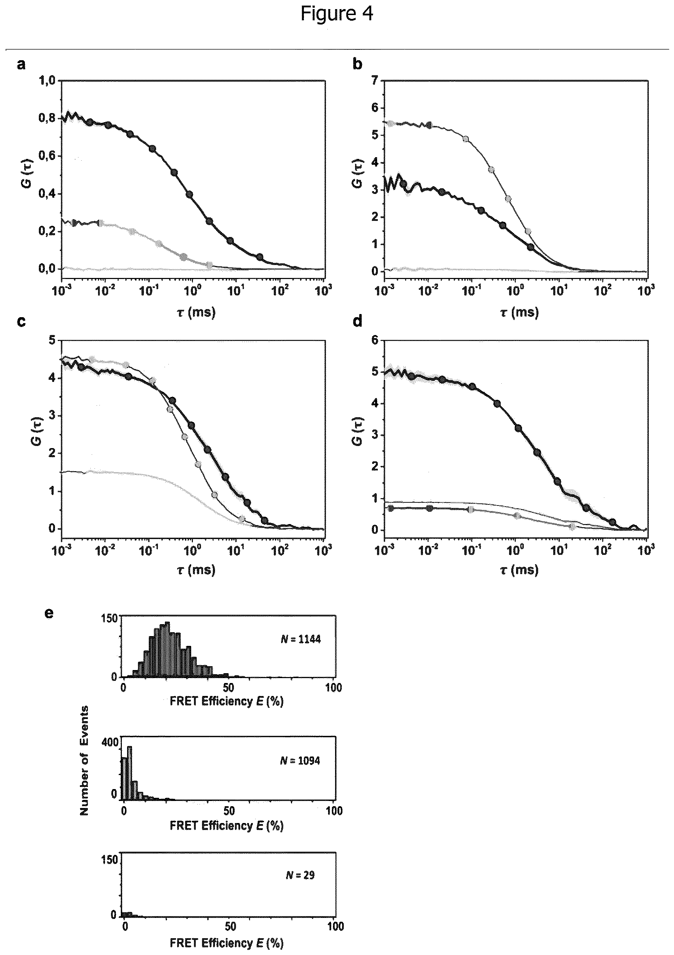

**Figure 4** shows the single-molecule interaction of PSM$\alpha$3_Atto647N with different $\alpha$-synuclein ($\alpha$S) species by FCCS and smFRET. (a-d) Representative auto-correlation curves for $\alpha$S (light grey circles) and PSM$\alpha$3_Atto647N (dark grey circles) and cross-correlation curves for interacting molecules (line without symbols). The amplitude (G) error is shown as faint colored area for the corresponding correlation curves. Samples contained (a) ~15 nM $\alpha$S monomer and ~15 nM PSM$\alpha$3_Atto647N, (b) 1 nM type A* and ~5 nM PSM$\alpha$3_Atto647N, (c) 1nM type B* oligomers (toxic oligomers) and ~5 nM PSM$\alpha$3_Atto647N or (d) ~5 nM fibrils and ~5 nM PSM$\alpha$3_Atto647N. (e) smFRET results of type B* oligomers (toxic oligomers) (top), fibrils (middle) and type A* oligomers (bottom). Representative intensity-calculated FRET efficiency histograms (right panels).

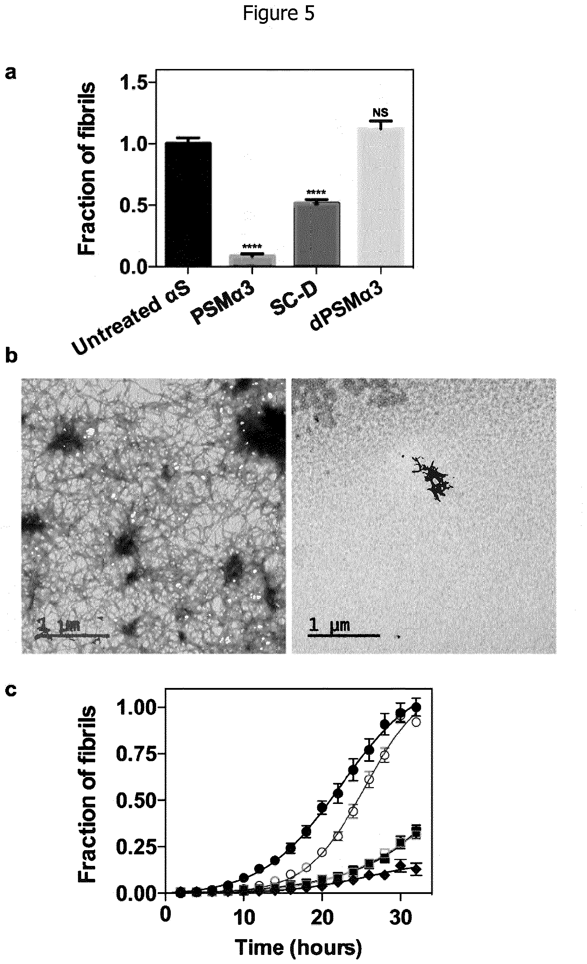

**Figure 5** shows the effect of PSM$\alpha$3 on the amyloid fibrillation of $\alpha$-synudein ($\alpha$S) *in vitro*. (A) Inhibition of $\alpha$-synudein amyloid aggregation followed by Th-T after 32 hours in absence or presence of equimolar concentrations of PSM$\alpha$3, Synuclean-D (SC-D) or dPSM$\alpha$3. (B) TEM micrographs of 70 $\mu$M of $\alpha$-synuclein aggregated for 32 hours in absence (left) or presence of equimolar concentration of PSM$\alpha$3 (right). (C) Titration of the inhibitory activity of PSM$\alpha$3 at different concentrations: 35 $\mu$M (diamonds), 14 $\mu$M (open squares), 7 $\mu$M (closed squares), 3.5 $\mu$M (open circles) and in absence of PSM$\alpha$3 (closed circles).

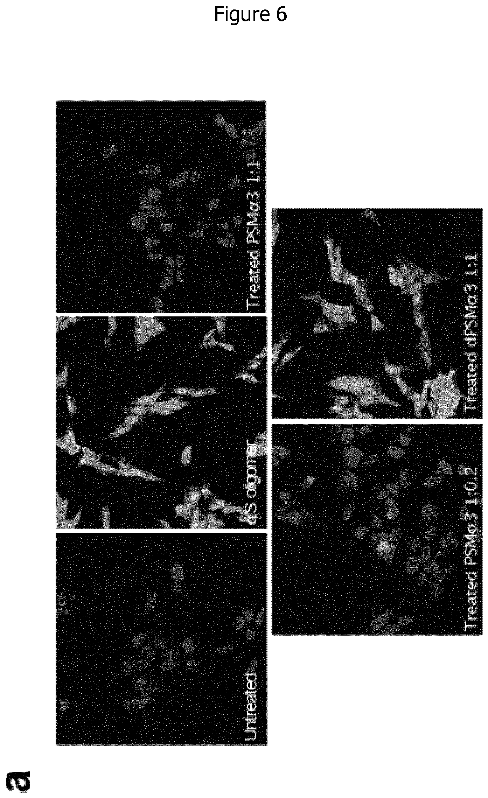

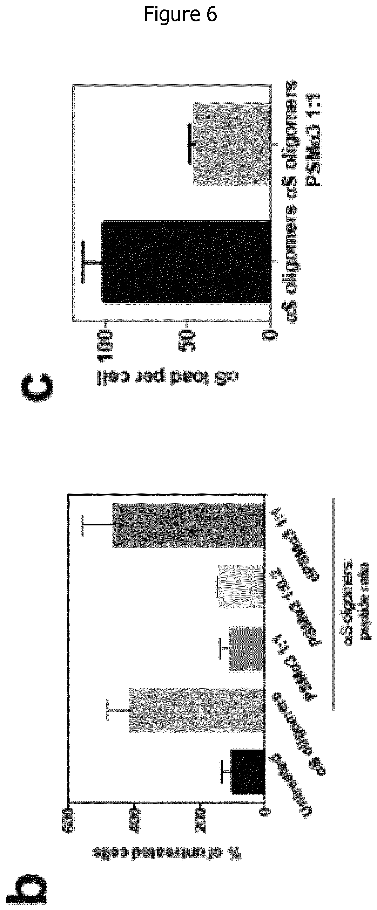

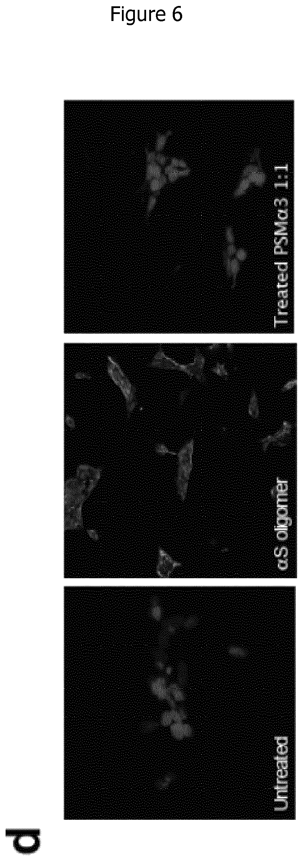

**Figure 6** shows the suppressive effect of PSM$\alpha$3 on the toxicity of $\alpha$-synudein ($\alpha$S) toxic oligomers (type B*) in cultured neuroblastoma SH-SY5Y cells. (a) Representative confocal images showing the intracellular levels of intracellular reactive oxygen species (ROS) in SH-SY5Y cells incubated with 10 $\mu$M of toxic $\alpha$-synuclein oligomer preincubated with different concentrations of PSM$\alpha$3 and dPSM$\alpha$3. (b) Quantification of the levels of intracellular ROS. (c) Quantification of the $\alpha$-synudein load per cell after the treatment with 10 $\mu$M of toxic $\alpha$-synudein oligomer pretreated with an equimolar concentration of PSM$\alpha$3. (d) Representative confocal images of the results shown in Fig. 6c.

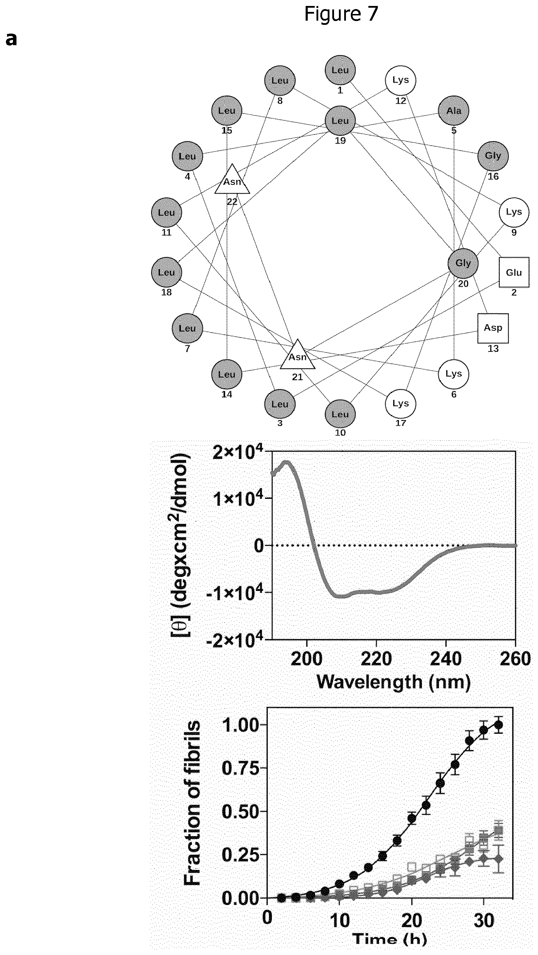

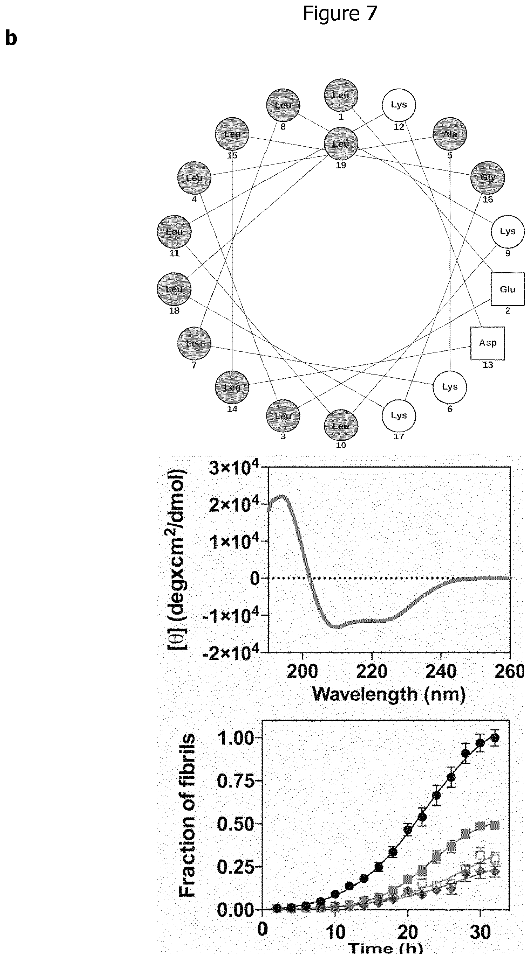

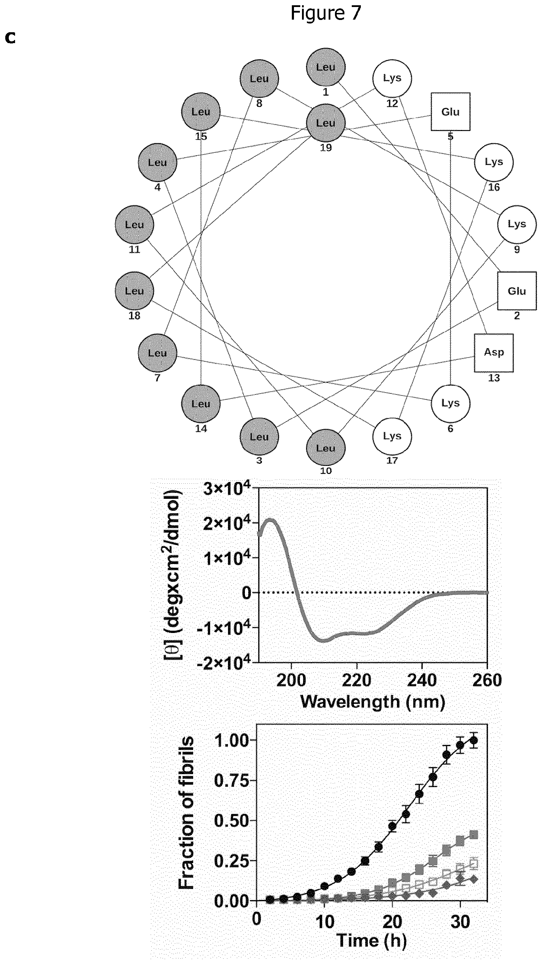

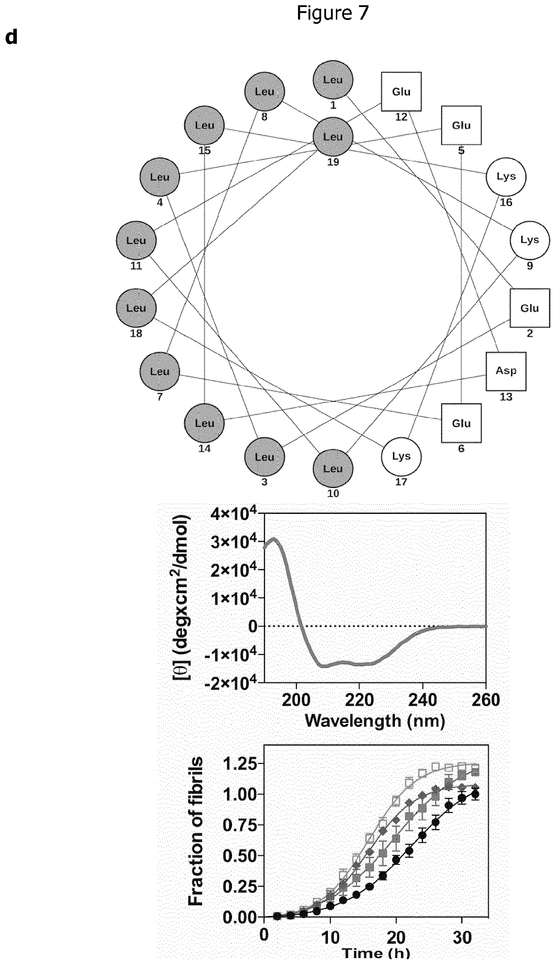

**Figure 7** shows the design of PSM$\alpha$3 variants which allow dissecting the molecular determinants of the interaction. (a-d) helical wheel (up), circular dichroism (CD) spectra (middle), titration of the inhibitory activity of the PSM$\alpha$3 variants at different concentrations (bottom): 35 $\mu$M (diamonds), 14 $\mu$M (open squares), 7 $\mu$M (closed squares) and in absence of PSM$\alpha$3 variants (closed circles). PSM$\alpha$3 variants: all_leu (a), all_leu19 (b), scaffold19 (c) and anionic_scaffold (d).

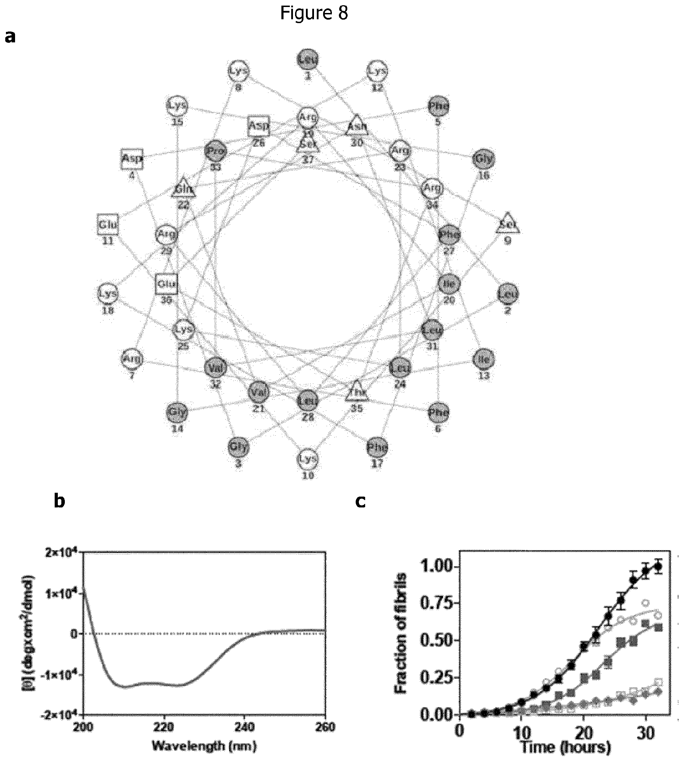

**Figure 8** shows the characterization of LL-37 and its capacity to inhibit $\alpha$-synudein ($\alpha$S) fibrillation. (A) Helical wheel projection of LL-37 sequence (circles: amino acid residues being colored depending on their hydrophilic/hydrophobic character; square box: proline). (B) Circular dichroism (CD) spectrum of LL-37 in PBS. (C) Titration of the inhibitory activity of LL-37 at different concentrations 35 $\mu$M (diamonds), 14 $\mu$M (open squares), 7 $\mu$M (closed squares) and in absence of LL-37 variants (closed circles).

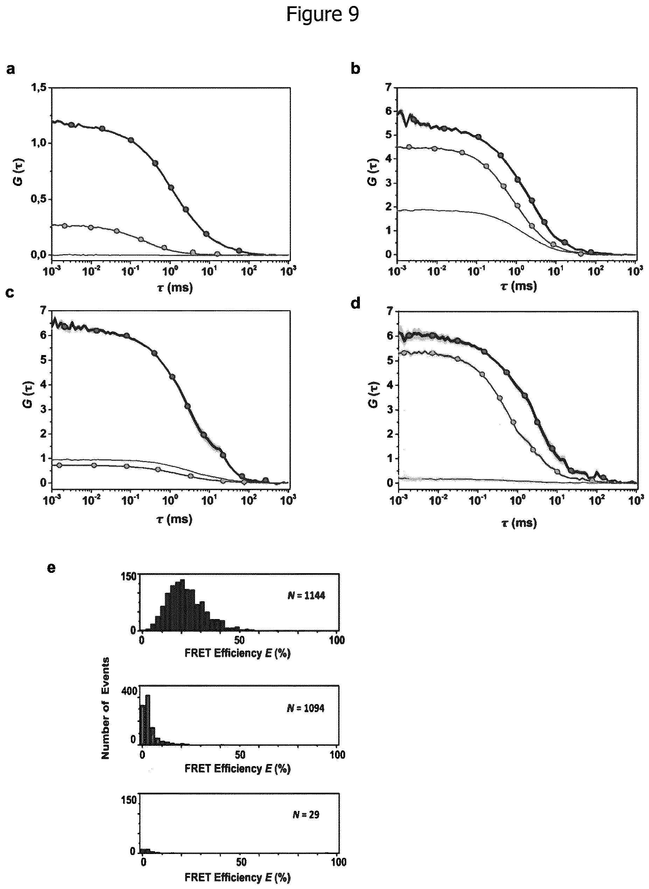

**Figure 9** shows the single-molecule interaction of LL-37_Atto647N with different $\alpha$-synuclein ($\alpha$S) species by FCCS and smFRET. (a-d) Representative auto-correlation curves for $\alpha$S (light grey circles) and LL-37_Atto647N (dark grey circles) and cross-correlation curves for interacting molecules (line without symbols. The amplitude (G) error is shown as faint colored area for the corresponding correlation curves. Samples contained (a) ~15 nM $\alpha$S monomer and ~15 nM LL-37_Atto647N, (b) 1 nM type B* (toxic oligomers) and ~5 nM LL-37_Atto647N, (c) 5 nM fibrils and ~5 nM LL-37_Atto647N or (d) ~1 nM type A* oligomers and ~5 nM LL-37_Atto647N. (e) smFRET results of type B* oligomers (toxic oligomers) (top), fibrils (middle) and type A* oligomers (bottom). Representative intensity-calculated FRET efficiency histograms.

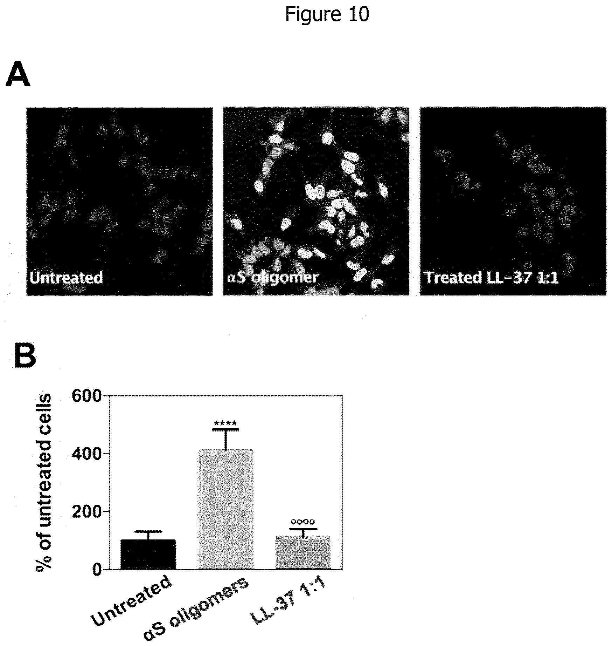

**Figure 10** shows representative confocal images of SH-SY5Y cells treated with 10 $\mu$M toxic $\alpha$-synudein oligomer in the presence of equimolar concentration of LL-37 (A), and the quantification of the intracellular ROS of this experiment (B).

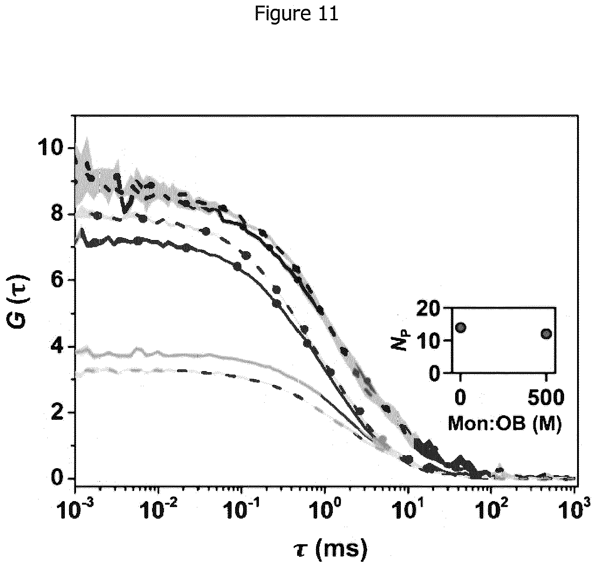

**Figure 11** shows the selectivity for the $\alpha$-synudein ($\alpha$S) type B* oligomers (toxic oligomers) vs monomeric $\alpha$S, determined through the single-molecule interaction of PSM$\alpha$3_Atto647N with $\alpha$-synudein ($\alpha$S) type B* oligomers (toxic oligomers) in the presences or absence of by FCCS. Auto-correlation curves ($\alpha$S: light grey circles, PSM$\alpha$3: dark grey circles) and cross-correlation curve for the interacting molecules (line without symbol) obtained in samples containing ~1 nM $\alpha$S type B* oligomers and ~2 nM PMS$\alpha$3 in the absence (solid lines) or presence (dashed lines) of a 500-molar excess of unlabeled monomer with respect to the particle concentration of oligomers. The inset shows the number of bound peptides (NP) per aggregate in both conditions.

## EXAMPLES

**Example 1:** $\alpha$-synudein expression and purification

Human $\alpha$-synudein can be expressed and purified as previously described in Pujols et al., 2018. Escherichia coli BL21 (DE3) cells harboring a pET21a plasmid encoding the $\alpha$-synudein gene (NCBI Gene ID: 6622) are grown in LB medium supplemented with 100 $\mu$M/mL ampicillin. Protein expression is induced at an optical density of 0.8 (600 nm) for 4 hours. Cells are harvested by centrifugation at washed up by resuspension and centrifugation in PBS (Phosphate buffered saline; 137 mM NaCl, 2.7 mM KCl, 10 mM $Na_2HPO_4$, 1.8 mM $KH_2PO_4$, pH 7.4, in water). Next, pellets are resuspended in 50 mL per culture liter in lysis buffer (50 mM Tris pH 8, 150 mM NaCl, 1 $\mu$g/mL pepstatin, 20 $\mu$g/mL aprotinin, 1 mM benzamidine, 1 mM PMSF, 1 mM EDTA and 0.25 mg/mL lysozyme) and sonicated using a sonicator (e.g., LabSonic®U sonicator, B. Braun Biotech International, Melsungen, Hessen, Germany). Samples are boiled for 10 minutes at 95°C and centrifuged at 20 000g at 4°C for 40 minutes. The soluble fraction is treated with 136 $\mu$L/mL of 10% w/v streptomycin sulfate and 228 $\mu$L/mL of pure acetic acid. Upon centrifugation, soluble extracts are fractionated by adding 1:1 of saturated ammonium sulfate and resuspending the insoluble fraction with 50% ammonium sulfate. The pellet is resuspended in 100 mM pH 8 ammonium acetate (5 mL per culture liter) and pure EtOH 1:1 (v/v) and harvested by centrifugation. The insoluble fraction is resuspended in 20 mM pH 8 Tris, filtered with a 0.22 $\mu$m filter and loaded into an anion exchange column (e.g., HiTrap Q HP coupled to an ÄKTA purifier high performance liquid chromatography system). $\alpha$-synuclein monomers are eluted using a using a step gradient: Step 1: 0%-20% buffer B, 5 column volumes (cv); Step 2: 20%-45% buffer B, 11 cv; Step 3: 100% buffer B, 5 cv. The $\alpha$-synuclein-containing fractions are collected and dialyzed against 5 L ammonium acetate 50 mM in two steps; 4 hours and overnight. Finally, protein purity is analyzed using 15% SDS-PAGE. The purest fractions are lyophilized and stored at -80°C. For the experiments, lyophilized aliquots of $\alpha$-synudein monomer are resuspended to a final concentration of 210 $\mu$M using PBS and filtered using 0.22 $\mu$m filters. $\alpha$-synudein concentration is determined by measuring the absorbance at 280 nm and using the extinction coefficient 5960 $M^{-1}$ $cm^{-1}$.

**Example 2:** $\alpha$-synudein and peptide labeling

In order to fluorescent-label $\alpha$-synuclein, the V122C variant of $\alpha$-synudein is employed which carries a single cysteine at position 122. This variant is expressed and purified as described above for the wild-type protein. The labeling is performed using maleimide-modified fluorescent dye according to manufacturer's instructions (e.g., maleimide-modified Alexa Fluor 488 dyes, Invitrogen, Carlsbad, CA, USA). Labeled $\alpha$-synudein is subsequently purified using a desalting column (e.g., P10 desalting column, GE Healthcare, Waukesha, WI, USA), flash-frozen with liquid nitrogen and stored at -80°C until assayed. For the peptide labeling, two PSM$\alpha$3 and LL-37 variants are designed containing a single cysteine in the N-terminal. These variants are incubated with maleimide-modified fluorescent dye (e.g., maleimide-modified Atto647N, ATTO-TEC, Siegen, Germany) for 16 hours at 4°C according to manufacturer's instructions. The labeled peptide is purified in two steps using a polyacrylamide desalting column (Thermo scientific, Waltham, MA, USA), flash-frozen with liquid nitrogen and stored at -80°C until assayed.

**Example 3:** Preparation of toxic α-synudein oligomers

6 mg aliquots of lyophilized purified α-synudein in PBS are prepared as described in example 1, each is resuspended with PBS to a final concentration of 800 μM, filtered through a 0.22 μm filter and incubated 20 to 24 hours at 37°C and without agitation. Fibrils formed during the incubation are removed by ultracentrifugation at 288 000 g (e.g., in a SW55Ti Beckman rotor). Monomeric α-synudein is removed from the supernatant obtained after ultracentrifugation by four consecutive cycles of filtration through 100 kDa centrifuge filters (e.g., from Merck, Darmstadt, Germany). As an example, 500 μL of the supernatant obtained after ultracentrifugation are placed in a centrifuge filter and centrifuged 2 minutes at 9300 g. The remaining unfiltered sample (around 100 μL) and 400 μL of fresh PBS are transferred to the next filter. The remaining unfiltered fraction after four cycles contains the toxic α-synudein oligomers which are kept at room temperature and must be used within three days. The final oligomer concentration is determined by measuring the absorbance at 280 nm and using the extinction coefficient 5960 $M^{-1}$ $cm^{-1}$.

**Example 4:** Preparation of non-toxic α-synudein oligomers

210 μM of purified α-synudein in PBS is prepared as described in example 1 and incubated with ten molar equivalents of (-)-epigallocatechin-3-gallate (EGCG) (e.g., from Merck, Darmstadt, Germany) for 48 hours at 37°C. After the incubation, monomeric protein is removed by six consecutive cycles of filtration through 100 kDa centrifuge filters (e.g. from Merck, Darmstadt, Germany). The final oligomer concentration is determined by measuring the absorbance at 280 nm and using the extinction coefficient 5960 $M^{-1}$ $cm^{-1}$.

**Example 5:** Preparation of α-synudein fibrils

A 96 well microtiter plate is prepared to contain, in each well, a Teflon polyball (3.175 mm in diameter, e.g., from Polysciences Europe GmbH, Eppelheim, Germany) and 150 μl of 70 μM α-synudein in PBS (prepared from diluting the 0.22 μm-filtered 210 μM α-synudein solution described in example 1). Said microtiter plate is incubated for 32 hours at 37°C while shaking (100rpm) on an orbital culture shaker (e.g., Max-Q 4000 Thermo Scientific, Waltham, MA, USA) so as to form α-synudein fibrils. The non-reacted protein and small non-fibrillar species that could be formed during the aggregation reaction were removed from the sample by 3 consecutive steps of centrifugation and resuspension of the precipitated fraction in PBS buffer at pH 7.4. Fibrillar samples are stored at room temperature until assayed.

**Example 6:** α-synudein aggregation kinetics assay

α-synudein aggregation can be monitored as described in Pujols et al., 2018. Specifically, inhibition of *in vitro* α-synudein aggregation at equimolar concentrations of α-synudein and the tested inhibitor can be determined as follows: A 96 well microtiter plate is prepared to contain, in each well, a Teflon polyball (3.175 mm in diameter, e.g., from Polysciences Europe GmbH, Eppelheim, Germany) and 150 μl of PBS containing 70 μM α-synudein (prepared from diluting the 0.22 μm-filtered 210 μM α-synudein solution described in example 1), 40 μM thioflavin-T and 70 μM of the tested inhibitor. 150 μl/well of PBS containing 70 μM α-synudein and 40 μM thioflavin-T is used as a control. Said microtiter plate is incubated for at 37°C while shaking (100rpm) on an orbital culture shaker (e.g., Max-Q 4000 Thermo Scientific, Waltham, MA, USA) - which induces for the formation of α-synudein fibrils. Every two hours, the fluorescence intensity is measured (e.g. using a Victor3.0 Multilabel Reader, PerkinElmer Waltham, MA, USA) by exciting the mixtures at 430-450 nm and measuring the emission intensity at 480-510 nm. End-point measures are done after 32 hours of incubation.

The microtiter plate should be from an opaque material (e.g. black plastic) to avoid interference by the fluorescent in adjacent wells during fluorescence reading. Parallel wells (e.g. triplicates) are prepared for each tested inhibitor and the control. For the measurement of each time interval, the measured fluorescence intensity of the respective parallel wells is averaged and then normalized to the maximum fluorescence of the control (a-synuclein without inhibitor) and fitted to the Finke-Watzky curve, accounting for fibril elongation and secondary nucleation.

$$\alpha = 1 - \frac{1}{k_b(e^{k_a t} - 1) + 1}$$

kb and ka represent the homogeneous nucleation rate constant and the secondary rate constant (Crespo et al., 2016). Comparison of the resulting curves (kinetic curves) of the tested inhibitors and the controls allows for determining whether there is inhibition of α-synudein aggregation.

Inhibition of *in vitro* α-synudein aggregation by a tested inhibitor at an excess of α-synuclein can be determined by the same procedure as described above, apart from using less than 70 $\mu$M of the tested inhibitor when preparing the microtiter plate, for example 25 $\mu$M (for a 2-fold excess of $\alpha$-synudein), 17.4 $\mu$M (for a 4-fold excess of $\alpha$-synudein), 11.67 $\mu$M (for an about 6-fold excess of $\alpha$-synudein), 8.75 $\mu$M (for a 8-fold excess of $\alpha$-synudein), 7 $\mu$M (for a 10-fold excess of $\alpha$-synudein), or 4.67 $\mu$M (for an about 15-fold excess of $\alpha$-synudein).

**Example 7:** Inhibition of the toxic $\alpha$-synudein oligomer induced increase in the level of intracellular reactive oxygen species

A stock solution of 80 $\mu$M toxic $\alpha$-synudein oligomer (prepared as described in example 3) and a stock solution of 80 $\mu$M toxic $\alpha$-synudein oligomer and 80 $\mu$M of the tested inhibitor are prepared in PBS and incubated for 15 min at 20°C before being added to cell-containing wells as described below.

Human SH-SY5Y neuroblastoma cells (ATCC, #CRL-2266) cultured in growth medium (Eagle's Minimum Essential Medium (EMEM) supplemented with 10% fetal serum albumin (FBS)) are seeded onto a glass slide mounted with 8 removable wells (e.g., Nunc Lab-Tek II Chamber Slide System, #154941) with 0.5 mL cells / well at $0.5 \times 10^6$ cells/mL in growth medium for 24h at 37°C and 5% $CO_2$. Then, stock solutions of toxic $\alpha$-synudein oligomer or toxic $\alpha$-synudein oligomer + inhibitor are added to the cell-containing wells to a final concentration of 10 $\mu$M toxic $\alpha$-synudein oligomer and the wells are incubated for 15 minutes at 37°C and 5% $CO_2$.

Then, a fluorogenic reagent for oxidative stress detection in live cells (e.g., a CellRox® reagent from Invitrogen, Carlsbad, USA, such as CellRox® Green) is added (final concentration of CellRox® Green 5 $\mu$M). After incubation for 30 minutes at 37°C and 5% $CO_2$, the cells are washed with PBS, fixed with 3.7% paraformaldehyde (PFA) in PBS for 30 min, and washed again with PBS. The intracellular fluorescence of the treated SH-SY5Y cells is then analyzed with a confocal microscope (e.g. a Leica TCS SP5, Leica Microsystems, Wetzlar, Germany, with a HCX PL APO 63 $\times$ 1.4 oil immersion objective) equipped with a camera, using wave lengths for excitation and detection which are suitable for the used oxidative stress detection agent (for CellRox® Green: excitation at 488 nm and detection at 515-560 nm). Intracellular fluorescence was measured by drawing region of interest (ROI) around the cells and analyzing the fluorescence intensity. ROS fluorescence are calculated from mean of 100 cells.

**Example 8:** Inhibition of toxic $\alpha$-synudein oligomers interaction with cells

A stock solution of 80 $\mu$M toxic $\alpha$-synudein oligomer (prepared as described in example 3) and a stock solution of 80 $\mu$M toxic $\alpha$-synudein oligomer and 80 $\mu$M of the tested inhibitor are prepared and incubated for 15 min at 20°C before being added to cell-containing wells as described below.

Human SH-SY5Y neuroblastoma cells (ATCC, #CRL-2266) cultured in growth medium (Eagle's Minimum Essential Medium (EMEM) supplemented with 10% fetal serum albumin (FBS)) are seeded onto a glass slide mounted with 8 removable wells (e.g., Nunc Lab-Tek II Chamber Slide System, #154941) with 0.5 mL cells / well at $0.35 \times 10^6$ cells/mL in growth medium. After 24h incubation at 37°C and 5% $CO_2$, the stock solutions of toxic $\alpha$-synudein oligomer or toxic $\alpha$-synudein oligomer + inhibitor are added to the cell-containing wells to a final concentration of 10 $\mu$M toxic $\alpha$-synuclein oligomer, and the slides are incubated for 45 minutes at 37°C and 5% $CO_2$.

Then, cells are washed with PBS to remove not internalized oligomers, and fixed with 3.7% paraformaldehyde (PFA) in PBS for 30 minutes at room temperature. Next, to permeabilize membranes, cells are washed with PBS containing 0.1% Triton X-100 for 10 min. Cells are blocked with 5% BSA in PBS and incubated with a suitable working solution of a primary anti-$\alpha$-synudein antibody (e.g. rabbit polyclonal anti-$\alpha$-synuclein antibody, Abcam, Cambridge, UK, 1:200 diluted in 5% BSA in PBS) overnight at 4°C, and then with a suitable working solution of a fluorophore-labeled secondary antibody (e.g. anti-rabbit secondary antibody, conjugated with Alexa Fluor 488 from Molecular Probes, 1:1000 diluted in 5% BSA in PBS) for 1h at room temperature. Cells are washed with PBS and cell nuclei are stained using a suitable dye (e.g. Hoechst 33342 at a concentration of 0.5 $\mu$g/mL for 5 min). Images of labeled intracellular $\alpha$-synudein are obtained using a confocal microscope (e.g. a Leica TCS SP5, Leica Microsystems, Wetzlar, Germany, with a HCX PL APO 63 $\times$ 1.4 oil immersion objective) equipped with a camera, and using wave lengths for excitation and detection which are suitable for the used fluorophore-labeled secondary antibody and cell nuclei stain (for Alexa Fluor 488 labeled antibody and Hoechst 33342: excitation at 488 nm and 350 nm, and detection at 515-560 nm).

**Example 9:** dcFCCS analysis of $\alpha$-synudein binding

The relative binding affinities of inhibitors of the present disclosure to toxic $\alpha$-synuclein oligomers, $\alpha$-synudein fibrils, and monomeric $\alpha$-synudein can be determined via single-molecule Förster resonance energy transfer (dcFCCS) as follows.

Dual-Colour FCCS (dcFCCS) experiments are performed on a Time-Resolved Fluorescence confocal microscope (e.g., MT200 from PicoQuant, Berlin, Germany) with a Time-Correlated Single Photon Counting (TCSPC) unit. Pulsed Interleaved Excitation (PIE) is used at laser powers of 6 $\mu$W (481 nm) and 5 $\mu$W (637 nm) measured after the dichroic mirror for optimal count rates while avoiding photobleaching and saturation. The coverslip is placed directly on the immersion water on top of the objective (e.g., Super Apochromat 60x NA 1.2 objective with a correction collar). The dichroic 488/640 nm is chosen as the main beam splitter. A 50 $\mu$m pinole is used. The emission light beam is split by a 50/50 beamsplitter into 2 detection paths. Bandpass emission filters of 520/35 for the donor dye and 690/70 for the acceptor dye are used before the detectors. Single Photon Avalanche Diodes (SPADs) serve as detectors. Each measurement has an acquisition time of 1 to 5 minutes. Autocorrelation and cross-correlation are carried using a suitable software (e.g., SymphoTime64 from PicoQuant, Berlin, Germany). The software is also used for fitting the FCS and FCCS data as well as for a PIE-FRET analysis of the data from which the FRET efficiency (E) is calculated.

**Example 10:** smFRET analysis of $\alpha$-synudein binding

The binding of inhibitors of the present disclosure to toxic $\alpha$-synudein oligomers, $\alpha$-synuclein fibrils, and monomeric $\alpha$-synudein can be determined via Förster resonance energy transfer (smFRET) as follows.

For single-molecule FRET, an acceptor (red dye) direct excitation lower threshold based on the mean intensity of the time trace (IA,mean+ $2\times\sigma$) is used to filter out those events without an active acceptor molecule. To further select those events arising from $\alpha$-synudein aggregates, a burst selection intensity threshold of 100 photons is used. Burstwise FRET efficiency (E) are calculated as given by E= $F_{A,IE}/(F_D+F_{A,DE})$ where $F_D$ is the fluorescence intensity in the donor (green) channel, $F_{A,IE}$ is the fluorescence intensity in the acceptor (red) channel through indirect excitation and $F_{A,DE}$ is the fluorescence intensity in the acceptor (red) channel after direct excitation by PIE pulse (Kruger et al., 2017).

**Example 11:** Far-UV circular dichroism (CD) analysis

Peptides are diluted to 15 $\mu$M in water. Far-UV CD spectra are measured on a respective spectrometer (e.g., Jasco J-815 CD spectrometer, Halifax, Nova Scotia, Canada). The CD signal is measured from 260 nm to 190 nm at 0.2 nm intervals, 1 nm bandwidth, 1 second of response time and a scan speed of 100 nm/min on a 0.1 cm quartz cell. 10 accumulations rerecorded for each measurement. In the case of LL-37, CD spectra are recorded in PBS pH 7.4 since structural differences are reported between water and saline solvents (Johansson et al., 1998). Thus, the spectra are recorded from 260 nm to 200 nm to avoid the interference of the saline buffer in the range 190 nm to 200 nm with the same parameters as indicated above.

**Example 12:** Transmission electron microscopy

For electron microscopy analyses, end-point samples from the aggregation assay are sonicated for 5 minutes at minimum intensity in an ultrasonic bath (e.g., VWR ultrasonic cleaner), placed onto carbon-coated copper grids and allowed to absorb for 5 minutes. The grids are then washed with distilled water and negative-stained with 2% (w/v) uranyl acetate for 1 min. Finally, the excess of uranyl acetate is absorbed using ashless filter paper and the grids are left to air-dry for 15 minutes. For detection a transmission electron microscope (e.g., TEM JEM-1400 from JEOL, Peabody, MA, USA) is used operating at accelerating voltage of 120kV.

The following examples describe experiments wherein the techniques and the specific exemplified materials and devices described in examples 1-12 were used.

**Example 13: Characterization of binding to different $\alpha$-synudein species**

$\alpha$-synudein species, monomer, non-toxic oligomer (type A*), toxic oligomer (type B*) and fibrils, were prepared as described in examples 1-5. The interaction of PSM$\alpha$3 or LL-37 (each labeled with Atto647N) with said $\alpha$-synudein species was examined by dcFCCS and smFRET according to examples 9 and 10.

Both PSM$\alpha$3 and LL-37 were found to interact with, toxic $\alpha$-synudein oligomer and fibrils resulting in an increase in the cross-correlation signal and numerous smFRET events. (see Fig. 4c, d and e, Fig. 9c, d and e) but not with $\alpha$-synudein monomer or non-toxic $\alpha$-synudein (see Fig. 4a, b and e, Fig. 9a, b and e). These results show that PSM$\alpha$3 and LL-37 specifically bound to the pathogenic $\alpha$-synudein species.

**Example 14: PSMa3 and LL-37 block $\alpha$-synudein amyloid aggregation**

Monomeric $\alpha$-synudein was prepared as described as described in example 1. $\alpha$-synuclein aggregation kinetics were monitored in absence and presence of the assayed peptides according to example 6. End-point (32h of incubation) aggregation reactions of the coincubations with PSM$\alpha$3 were also analyzed by means of TEM as described in example 12.

PSM$\alpha$3 and LL-37 were reported to inhibit $\alpha$-synudein aggregation at equimolar and substoichiometric concentrations (see Fig. 5a-c and Fig. 8 c), thus demonstrating that PSM$\alpha$3 and LL-37 interaction with pathogenic $\alpha$-synudein

species blocks the progression of the amyloid reaction (aggregation).

**Example 15: PSMa3 and LL-37 ameliorate the cellular toxicity associated $\alpha$-synudein toxic oligomers**

Toxic oligomer (type B*) and fibrils, were prepared as described in example 3. PSM$\alpha$3 and LL-37 inhibition of the oligomer associated toxicity and oligomer interaction with cells were assessed as described in examples 7-8, respectively. PSM$\alpha$3 and LL-37 reduce the generation of reactive oxygen species when coincubated with $\alpha$-synudein toxic oligomers (see Fig. 6a-b and Fig. 10a-b). PSM$\alpha$3 also reduces the interaction of $\alpha$-synudein toxic oligomers with cells (see Fig. 6c-d). These results indicate that the presence of PSM$\alpha$3 and LL-37 have detoxifying activities when coincubated with $\alpha$-synudein toxic oligomers.

**Characteristics of exemplary peptides**

|  | SEQ ID NO | helical hydrophobic moment | AGADIR value | net charge |
|---|---|---|---|---|
| **PSM$\alpha$3** | 2 | 0.56 | 2.65 | 2 |
| **dPSM$\alpha$3** | 3 | N/A | 0.40 | 1 |
| **All_Leu** | 4 | 0.58 | 66.14 | 2 |
| **All_Leu19** | 5 | 0.70 | 65.17 | 2 |
| **Scaffold_19** | 6 | 0.77 | 77.68 | 2 |
| **Anionic Scaffold** | 7 | 0.63 | 78.26 | -2 |
| **LL-37** | 8 | 0.52 | 5.10 | 6 |
| **$\beta$-synuclein (region)** | 9 | 0.24 | 1.13 | 2 |

**SEQUENCES**

| designation | sequence | SEQ ID NO |
|---|---|---|
| full-length human wild-type $\alpha$-synuclein | MDVFMKGLSKAKEGVVAAAEKTKQGVAEAAGKTKEGVLYV GSKTKEGVVHGVATVAEKTKEQVTNVGGAVVTGVTAVAQK TVEGAGSIAAATGFVKKDQLGKNEEGAPQEGILEDMPVDP DNEAYEMPSEEGYQDYEPEA | 1 |
| PSM$\alpha$3 | MEFVAKLFKFFKDLLGKFLGNN | 2 |
| dPSM$\alpha$3 | MEFVAKLFPFPKDLLGKFLGNN | 3 |
| All-Leu | LELLAKLLKLLKDLLGKLLGNN | 4 |
| All-Leu-19 | LELLAKLLKLLKDLLGKLL | 5 |
| Scaffold-19 | LELLEKLLKLLKDLLKKLL | 6 |
| Anionic Scaffold | LELLEELLKLLEDLLKKLL | 7 |
| LL-37 | LLGDFFRKSKEKIGKGFKRIVQRLKDFLRNLVPRTES | 8 |
| $\beta$-synuclein (region) | MDVFMKGLSMAKEGVVAAAEKTKQGVTEAAEKTKEGVLY VGSKTREGVVQGVASVAEKTKEQASHLGGAVFS | 9 |

**LIST OF REFERENCES**

Bacia et al., 2007, Nat Protoc, 2(11), 2842-2856

Benskey et al., 2016, J Neurochem, 137:331-359

Chen et al., 2015, Proc Natl Acad Sci U S A 112(16): E1994-2003

Chen et al., 2018, Methods Mol Biol, 1779: 45-60

Cremades et al., 2012, Cell, 149, 1048-1059

Crespo et al., 2016, J Biol Chem 291(4): 2018-2032

Csáki et al., 2018, Expert Rev Mol Diagn, 18(3), 279-296

Eisenberg et al., 1982, Nature, 299, 371-374

Froula et al., 2019, J Biol Chem, 294, 10392-10406

Fusco et al., 2017, Science, 358, 1440-1443

Gautier et al., 2008, Bioinformatics, 24(18), 2101-2102

Honarmand et al., 2019, PLoS One, 14(6), e0217801

Horrocks et al., 2016, ACS Chem Neurosci, 7(3), 399-406

Johansson et al., 1998, J Biol Chem, 273(6), 3718-3724

Kruger et al., 2017, Biophys J, 113, 1311-1320

Marinelli et al., 2016, Sci Rep, 6, 34552

Munoz et al., 1994, J Mol Bio., 245(3), 297-308

Nath et al., 2010, Biophys J, 98(7), 1302-1311

Nguyen et al., 2015, Sensors (Basel), 15(5), 10481-510

Peña-Diaz et al., 2019, Front Mol Neurosci, 12, 306

Pujols et al., 2018, Proc Natl Acad Sci U S A, 115(41), 10481-10486

Pujols et al., 2020, Trends Mol Med, 26(4), 408-421

Shahnawaz et al., 2017, JAMA Neurol, 74(2), 163-172

Singh et al., 2006, Anal Chem, 78(6), 2009-2018

Spillantini et al., 1999, Parkinsonism Relat Disord, 5(4), 157-162

Sun et al., 2017, Sensors and Actuators B: Chemical, 245, 87-94

Tokuda et al., 2010, Neurology, 75(20), 1766-1772

Tsigelny et al., 2015, ACS Chem Neurosci, 6(3), 403-416

Weng et al., 2015, Methods Mol Biol, 1278, 341-352

Winner et al., 2011, Proc Natl Acad Sci U S A, 108 (10), 4194-4199

Zamyatnin et al., 2006, Nucleic Acids Res, 34(Database issue), D261-D266

## Claims

1. An inhibitor of α-synudein aggregation for use in the treatment and prophylaxis of a synucleinopathy in a mammalian subject, preferably a human, wherein the inhibitor comprises a peptide of at least 10 consecutive amino acid residues, said peptide having:

   - an amphipathic α-helical structure as defined by an AGADIR value of 1 or greater and by a helical hydrophobic moment of 0.1 or greater, and
   - a net charge that is 0 or positive at pH 7.4.

2. An *in-vitro* method for detecting α-synudein aggregates in a biological sample of a mammalian subject, optionally a human subject, wherein the method comprises contacting an inhibitor of α-synudein aggregation with the sample and then detecting the binding of α-synudein aggregates comprised in the sample to the inhibitor, wherein the inhibitor comprises a peptide of at least 10 consecutive amino acid residues, said peptide having:

   - an amphipathic α-helical structure as defined by an AGADIR value of 1 or greater and by a helical hydrophobic moment of 0.1 or greater, and
   - a net charge that is 0 or positive at pH 7.4.

3. The method of claim 2, wherein binding of α-synudein aggregates to the immobilized inhibitor is detected by a label-free technique for detecting the interaction of biomolecules; and
   optionally, wherein the inhibitor is immobilized on the sensor surface of a plasmonic sensor device, and binding of α-synudein aggregates to the immobilized inhibitor is detected by Surface Plasmon Resonance (SPR) spectroscopy, preferably by Localized Surface Plasmon Resonance (LSPR) spectroscopy.

4. The method of claim 2 or claim 3 that is a method of diagnosing a synucleinopathy in the mammalian subject, wherein the detected α-synudein aggregate level being higher than the α-synudein aggregate levels in healthy subjects of the same species indicates that the mammalian subject suffers from a synucleinopathy.

5. The method of any one of claims 2-4, wherein the biological sample is a sample of the cerebrospinal fluid of the subject.

6. A sensor of a plasmonic sensor device, the sensor carrying on its surface an immobilized inhibitor of α-synudein aggregation, wherein the inhibitor comprises a peptide of at least 10 consecutive amino acid residues, said peptide having:

- an amphipathic α-helical structure as defined by an AGADIR value of 1 or greater and by a helical hydrophobic moment of 0.1 or greater, and
- a net charge that is 0 or positive at pH 7.4;

optionally, wherein at least part of the surface of the sensor is formed by nanoparticles, and wherein the plasmonic sensor device is preferably a Localized Surface Plasmon Resonance (LSPR) spectrometer.

7. A kit for detecting α-synudein aggregates in a biological sample, wherein the kit comprises an inhibitor of α-synudein aggregation and a control sample comprising α-synudein aggregates, wherein the inhibitor comprises a peptide of at least 10 consecutive amino acid residues, said peptide having:

- an amphipathic α-helical structure as defined by an AGADIR value of 1 or greater and by a helical hydrophobic moment of 0.1 or greater, and
- a net charge that is 0 or positive at pH 7.4;

optionally, wherein the inhibitor is immobilized on the sensor surface of plasmonic sensor device, and wherein the plasmonic sensor device is preferably a Localized Surface Plasmon Resonance (LSPR) spectrometer.

8. An inhibitor of α-synudein aggregation comprising a peptide of at least 10 consecutive amino acid residues, said peptide having:

- an amphipathic α-helical structure **characterized by** an AGADIR value of 1 or greater and by a helical hydrophobic moment of 0.1 or greater, and
- a net charge that is 0 or positive at pH 7.4;

provided that the inhibitor is not a peptide having the amino acid sequence of SEQ ID NO:2 or SEQ ID NO:8 or SEQ ID NO:9.

9. A pharmaceutical composition comprising an inhibitor of α-synudein aggregation and at least one pharmaceutically acceptable carrier, wherein the inhibitor comprises a peptide of at least 10 consecutive amino acid residues, said peptide having:

- an amphipathic α-helical structure **characterized by** an AGADIR value of 1 or greater and by a helical hydrophobic moment of 0.1 or greater, and
- a net charge that is 0 or positive at pH 7.4.

10. An inhibitor of α-synudein aggregation for use in medicine, wherein the inhibitor comprises a peptide of at least 10 consecutive amino acid residues, said peptide having:

- an amphipathic α-helical structure **characterized by** an AGADIR value of 1 or greater and by a helical hydrophobic moment of 0.1 or greater, and
- a net charge that is 0 or positive at pH 7.4.

11. The inhibitor for use according to claim 1 or claim 10, or the *in-vitro* method of any one of claims 2-5, or the sensor of claim 6, or the kit of claim 7, or the inhibitor of claim 8, or the pharmaceutical composition of claim 9, wherein the amphipathic α-helical peptide is a peptide of at least 12, at least 15, at least 19, such as 10-100, 12-60, 15-50 or 19-40 consecutive amino acid residues.

12. The inhibitor for use according to any one of claims 1, 10 and 11, or the *in-vitro* method of any one of claims 2-5 and 11, or the sensor of claim 6 or claim 11, or the kit of claim 7 or claim 11, or the inhibitor of claim 8 or claim 11, or the pharmaceutical composition of claim 9 or claim 11, wherein the net charge of the amphipathic α-helical peptide at pH 7.4 is + 0.1 or greater, +0.5 or greater, or +1 or greater, or is in the range of from 0 to +10, from 0 to +7, from 0 to +6, from 0.1 to +5, from 0.1 to +4, from 0.5 to +3, or from 0.5 to +2.5.

13. The inhibitor for use according to any one of claims 1 and 10-12, or the *in-vitro* method of any one of claims 2-5, 11 and 12, or the sensor of any one of claims 6, 11 and 12, or the kit of any one of claims 7, 11 and 12, or the inhibitor of any one of claims 8, 11 and 12, or the pharmaceutical composition of any one of claims 9, 11 and 12,

wherein the helical hydrophobic moment of the amphipathic $\alpha$-helical peptide is 0.2 or greater, or 0.3 or greater, or is in the range of 0.10-0.90, 0.15-0.80, 0.20-0.70, or 0.25-0.60.

14. The inhibitor for use according to any one of claims 1 and 10-13, or the *in-vitro* method of diagnosing a synucleinopathy according to any one of claims 4, 5, 11, 12 and 13, wherein:

(a) the synucleinopathy is selected from Parkinson's Disease, Dementia with Lewy Bodies, Multiple System Atrophy, Pure Autonomic Failure, Lewy Body Variant of Alzheimer's Disease and Neurodegeneration with Brain Iron Accumulation; or
(b) the synucleinopathy is familial; or
(c) the subject of the treatment or the prophylaxis presents multiplications of the gene encoding $\alpha$-synudein (*SNCA* gene), or expresses $\alpha$-synudein comprising at least one amino acid substitution selected from:

(xvi) a proline at position 30,
(xvii) a lysine at position 46,
(xviii) a glutamine at position 50,
(xix) an aspartate at position 51, and
(xx) a threonine, a valine or a glutamate at position 53;

the numbering of said amino acid positions being relative to the of full length $\alpha$-synudein as set forth in SEQ ID NO:1.

15. The inhibitor for use according to any one of claims 1 and 10-14, or the *in-vitro* method of any one of claims 2-5 and 11-14, or the sensor of any one of claims 6 and 11-13, or the kit of any one of claims 7 and 11-13, or the inhibitor of any one of claims 8 and 11-13, or the pharmaceutical composition of any one of claims 9, and 11-13, wherein the inhibitor comprises an amino acid sequence having at least 84%, at least 88, at least 90%, at least 94%, or at least 97% sequence identity to an amino acid sequence selected from SEQ ID NOs:2, 4, 5, 6 and 8.

## Figure 1

|  | Monomer | Oligomer type A* | Oligomer type B* | Fibrils |
|---|---|---|---|---|
| Number of monomers | 1 | ~30† | ~30† | >100† |
| Anionic character | -9 | ~-270† | ~-270† | >-900 |
| Relative hydrophobicity | Low | Low | High | Medium |
| Secondary structure | Disordered | Disordered | β-sheet | β-sheet |

## Figure 2

**A**

**B**

180°

**C**

Figure 3

c

d

# Figure 4

# Figure 5

Figure 6

# Figure 6

Figure 6

# Figure 7

a

# Figure 7

**b**

# Figure 7

C

## Figure 7

**d**

Figure 8

**a**

**b**

**c**

# Figure 9

Figure 10

**A**

**B**

Figure 11

Europäisches Patentamt
European Patent Office
Office européen des brevets

## EUROPEAN SEARCH REPORT

Application Number

EP 20 38 2658

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PARK J-Y ET AL: "[beta]-Synuclein Inhibits Formation of [alpha]-Synuclein Protofibrils: A Possible Therapeutic Strategy against Parkinson's Disease", BIOCHEMISTRY, vol. 42, no. 13, 1 April 2003 (2003-04-01), pages 3696-3700, XP055805468, DOI: 10.1021/bi020604a Retrieved from the Internet: URL:https://pubs.acs.org/doi/pdf/10.1021/bi020604a> | 1,9-14 | INV. G01N33/68 G01N33/543 C07K14/31 A61P25/28 A61K38/00 |
| A | * the whole document, in particular abstract * | 8 | |
| X | TAYEB-FLIGELMAN E ET AL: "Staphylococcus aureus PSM[alpha]3 Cross-[alpha] Fibril Polymorphism and Determinants of Cytotoxicity", STRUCTURE, vol. 28, no. 3, 6 January 2020 (2020-01-06), page 301, XP086074571, DOI: 10.1016/J.STR.2019.12.006 [retrieved on 2020-01-06] * table 1 * | 8,11-13, 15 | |

-/--

TECHNICAL FIELDS SEARCHED (IPC)

A61P
A61K
G01N
C12R
C07K

The present search report has been drawn up for all claims

| Place of search | Date of completion of the search | Examiner |
|---|---|---|
| Munich | 19 May 2021 | Weber, Peter |

EPO FORM 1503 03.82 (P04C01)

page 1 of 5

Europäisches
Patentamt

European
Patent Office

Office européen
des brevets

**EUROPEAN SEARCH REPORT**

Application Number

EP 20 38 2658

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZUGHAIER S M ET AL: "The Human Host Defense Peptide LL-37 Interacts with Neisseria meningitidis Capsular Polysaccharides and Inhibits Inflammatory Mediators Release", PLOS ONE, vol. 5, no. 10, 26 October 2010 (2010-10-26), page e13627, XP055805440, DOI: 10.1371/journal.pone.0013627 Retrieved from the Internet: URL:https://storage.googleapis.com/plos-co rpus-prod/10.1371/journal.pone.0013627/1/p one.0013627.pdf?X-Goog-Algorithm=GOOG4-RSA -SHA256&X-Goog-Credential=wombat-sa@plos-p rod.iam.gserviceaccount.com/20210518/auto/ storage/goog4_request&X-Goog-Date=20210518 T140036Z&X-Goog-Expires=3600&X-Goog-Signed Headers=ho> * table 1 * | 8,11-13, 15 | |
| X | GRONBERG A ET AL: "Treatment with LL-37 is safe and effective in enhancing healing of hard-to-heal venous leg ulcers: a randomized, placebo-controlled clinical trial", WOUND REPAIR AND REGENERATION, vol. 22, no. 5, 31 August 2014 (2014-08-31), pages 613-621, XP009517949, DOI: 10.1111/WRR.12211 [retrieved on 2014-07-17] * abstract * | 10-13,15 | TECHNICAL FIELDS SEARCHED (IPC) |
| X | WO 2018/005867 A2 (UNIV CALIFORNIA [US]) 4 January 2018 (2018-01-04) * the whole document, in particular claims 1, 8, 10, 13, 14, 16, 22 * | 1-15 | |

-/--

The present search report has been drawn up for all claims

| Place of search | Date of completion of the search | Examiner |
|---|---|---|
| Munich | 19 May 2021 | Weber, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 20 38 2658

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PACIOTTI S ET AL: "Are We Ready for Detecting [alpha]-Synuclein Prone to Aggregation in Patients? The Case of "Protein-Misfolding Cyclic Amplification" and "Real-Time Quaking-Induced Conversion" as Diagnostic Tools", FRONTIERS IN NEUROLOGY, vol. 9, 1 January 2018 (2018-01-01), page 415, XP055805458, DOI: 10.3389/fneur.2018.00415 Retrieved from the Internet: URL:https://www.ncbi.nlm.nih.gov/pmc/artic les/PMC5997809/pdf/fneur-09-00415.pdf> * abstract * | 2-7, 11-15 | |
| A | KE PU CHUN ET AL: "Implications of peptide assemblies in amyloid diseases", CHEMICAL SOCIETY REVIEWS, vol. 46, no. 21, 1 November 2017 (2017-11-01), pages 6492-6531, XP055805414, Retrieved from the Internet: URL:https://pubs.rsc.org/zh/content/articl epdf/2017/cs/c7cs00372b> * Section 4 * | 1-15 | |

-----

-----

-/--

TECHNICAL FIELDS
SEARCHED (IPC)

The present search report has been drawn up for all claims

| Place of search | Date of completion of the search | Examiner |
|---|---|---|
| Munich | 19 May 2021 | Weber, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 20 38 2658

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MARINELLI P ET AL: "Dissecting the contribution of Staphylococcus aureus [alpha]-phenol-soluble modulins to biofilm amyloid structure", SCIENTIFIC REPORTS, vol. 6, no. 34552, 6 October 2016 (2016-10-06), pages 1-13, XP055805296, DOI: 10.1038/srep34552 Retrieved from the Internet: URL:https://www.nature.com/articles/srep34552.pdf> * the whole document * | 1-15 | |
| T | SANTOS J ET AL: "a-Helical peptidic scaffolds to target a-synuclein pathogenic species with high affinity and selectivity", bioRxiv, 12 March 2021 (2021-03-12), pages 1-28, XP055805368, DOI: 10.1101/2021.03.12.435074 Retrieved from the Internet: URL:https://www.biorxiv.org/content/biorxiv/early/2021/03/13/2021.03.12.435074.full.pdf [retrieved on 2021-05-18] * the whole document *  -/-- | 1-15 | |

TECHNICAL FIELDS
SEARCHED      (IPC)

The present search report has been drawn up for all claims

| Place of search | Date of completion of the search | Examiner |
|---|---|---|
| Munich | 19 May 2021 | Weber, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt

European Patent Office

Office européen des brevets

# EUROPEAN SEARCH REPORT

Application Number

EP 20 38 2658

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | -& SANTOS J ET AL: "Supplementary Material: a-Helical peptidic scaffolds to target a-synuclein pathogenic species with high affinity and selectivity", bioRxiv, 12 March 2021 (2021-03-12), pages 1-11, XP055805376, Retrieved from the Internet: URL:https://www.biorxiv.org/content/10.1101/2021.03.12.435074v1.supplementary-material [retrieved on 2021-05-18] ----- | 1-15 | |

TECHNICAL FIELDS
SEARCHED       (IPC)

The present search report has been drawn up for all claims

| Place of search | Date of completion of the search | Examiner |
|---|---|---|
| Munich | 19 May 2021 | Weber, Peter |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 20 38 2658

This annex lists the patent family members relating to the patent documents cited in the above-mentioned European search report.
The members are as contained in the European Patent Office EDP file on
The European Patent Office is in no way liable for these particulars which are merely given for the purpose of information.

19-05-2021

| Patent document cited in search report | Publication date | Patent family member(s) | | Publication date |
|---|---|---|---|---|
| WO 2018005867 A2 | 04-01-2018 | CA | 3029676 A1 | 04-01-2018 |
| | | CN | 109641028 A | 16-04-2019 |
| | | EP | 3478311 A2 | 08-05-2019 |
| | | JP | 2019524685 A | 05-09-2019 |
| | | KR | 20190034214 A | 01-04-2019 |
| | | RU | 2019102202 A | 29-07-2020 |
| | | US | 2019241613 A1 | 08-08-2019 |
| | | US | 2021002329 A1 | 07-01-2021 |
| | | WO | 2018005867 A2 | 04-01-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BACIA et al.** *Nat Protoc,* 2007, vol. 2 (11), 2842-2856
- **BENSKEY et al.** *J Neurochem,* 2016, vol. 137, 331-359
- **CHEN et al.** *Proc Natl Acad Sci U S A,* 2015, vol. 112 (16), E1994-2003
- **CHEN et al.** *Methods Mol Biol,* 2018, vol. 1779, 45-60
- **CREMADES et al.** *Cell,* 2012, vol. 149, 1048-1059
- **CRESPO et al.** *J Biol Chem,* 2016, vol. 291 (4), 2018-2032
- **CSÁKI et al.** *Expert Rev Mol Diagn,* 2018, vol. 18 (3), 279-296
- **EISENBERG et al.** *Nature,* 1982, vol. 299, 371-374
- **FROULA et al.** *J Biol Chem,* 2019, vol. 294, 10392-10406
- **FUSCO et al.** *Science,* 2017, vol. 358, 1440-1443
- **GAUTIER et al.** *Bioinformatics,* 2008, vol. 24 (18), 2101-2102
- **HONARMAND et al.** *PLoS One,* 2019, vol. 14 (6), e0217801
- **HORROCKS et al.** *ACS Chem Neurosci,* 2016, vol. 7 (3), 399-406
- **JOHANSSON et al.** *J Biol Chem,* 1998, vol. 273 (6), 3718-3724
- **KRUGER et al.** *Biophys J,* 2017, vol. 113, 1311-1320
- **MARINELLI et al.** *Sci Rep,* 2016, vol. 6, 34552
- **MUNOZ et al.** *J Mol Bio.,* 1994, vol. 245 (3), 297-308
- **NATH et al.** *Biophys J,* 2010, vol. 98 (7), 1302-1311
- **NGUYEN et al.** *Sensors (Basel),* 2015, vol. 15 (5), 10481-510
- **PEÑA-DIAZ et al.** *Front Mol Neurosci,* 2019, vol. 12, 306
- **PUJOLS et al.** *Proc Natl Acad Sci U S A,* 2018, vol. 115 (41), 10481-10486
- **PUJOLS et al.** *Trends Mol Med,* 2020, vol. 26 (4), 408-421
- **SHAHNAWAZ et al.** *JAMA Neurol,* 2017, vol. 74 (2), 163-172
- **SINGH et al.** *Anal Chem,* 2006, vol. 78 (6), 2009-2018
- **SPILLANTINI et al.** *Parkinsonism Relat Disord,* 1999, vol. 5 (4), 157-162
- **SUN et al.** *Sensors and Actuators B: Chemical,* 2017, vol. 245, 87-94
- **TOKUDA et al.** *Neurology,* 2010, vol. 75 (20), 1766-1772
- **TSIGELNY et al.** *ACS Chem Neurosci,* 2015, vol. 6 (3), 403-416
- **WENG et al.** *Methods Mol Biol,* 2015, vol. 1278, 341-352
- **WINNER et al.** *Proc Natl Acad Sci U S A,* 2011, vol. 108 (10), 4194-4199
- **ZAMYATNIN et al.** *Nucleic Acids Res,* 2006, vol. 34, D261-D266